# EUROPEAN PATENT APPLICATION

(11) **EP 4 801 241 A1**
(43) Date of publication of application: **02.09.2026**
(21) Application number: 24882519.2
(22) Date of filing: 25.10.2024
(51) Int. Cl.: H10K 30/50, C07D 487/22, H10K 30/40, H10K 30/81, H10K 30/86, H10K 50/14, H10K 85/10, H10K 85/30, H10K 85/50

(54) **COMPOSITION, PREPARATION METHOD FOR COMPOSITION, AND PHOTOELECTRIC CONVERSION ELEMENT**

(30) Priority: 27.10.2023 JP 2023184761; 27.10.2023 JP 2023184756; 27.10.2023 JP 2023184750; 21.12.2023 JP 2023216294; 21.12.2023 JP 2023216296; 21.12.2023 JP 2023216299; 16.02.2024 JP 2024022244; 16.02.2024 JP 2024022251; 16.02.2024 JP 2024022246; 09.10.2024 JP 2024177315; 23.10.2024 JP 2024186707
(71) Applicant: Canon Kabushiki Kaisha, Tokyo 146-8501 (JP)
(72) Inventor: KATO Nanami, Tokyo 146-8501 (JP); NAKAMURA Nobuhiro, Tokyo 146-8501 (JP); OHSAWA Tatsuya, Tokyo 146-8501 (JP); NISHIDA Tsutomu, Tokyo 146-8501 (JP)
(74) Representative: TBK
(86) International application number: PCT/JP2024/038189
(87) International publication number: WO 2025/089411

(57) **Abstract**

Provided is such a composition that the dispersion stability of the composition and the photoelectric conversion efficiency of a photoelectric conversion element using the composition are improved. The composition is a composition including: a pigment that is a cyclic conjugated compound in which a plurality of pyrrole rings are bonded by conjugated bonds; a dispersant; a resin; and a solvent, wherein the dispersant is a calixarene compound represented by the following formula [A], and wherein a mass of the dispersant in the composition is 0.20 times or more with respect to a mass of the resin in the composition.

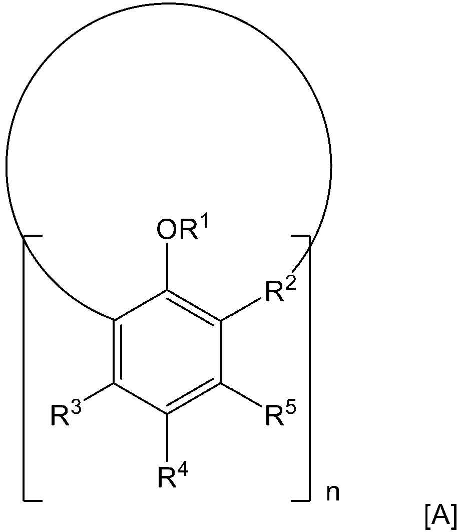

## Description

### [Technical Field]

The present invention relates to a composition, a method of preparing a composition, and a photoelectric conversion element.

### [Background Art]

In order to solve a problem of the depletion of fossil energy and a global environmental problem caused by the use of the fossil energy, investigations on a renewable and clean alternative energy source, such as solar energy, wind power, or water power, have been actively performed. In particular, an interest in a solar cell that directly changes sunlight into electrical energy has been increasing. The term "solar cell" as used herein means a battery that generates a current-voltage through utilization of a photovoltaic effect in which light energy is absorbed from sunlight to generate an electron and a hole.

Recently, an n-p diode-type silicon (Si) single crystal-based solar cell having a light energy conversion efficiency of more than 20% has been widely known, and has been actually used in solar power generation. However, the solar cell requires a high temperature treatment step and the price of a material itself is high, and hence there is a problem in that the cost per unit electric power is high. In addition, there is a problem with its supply property in terms of a silicon resource.

Meanwhile, a solar cell using an organic material (hereinafter also referred to as "organic solar cell") does not require the high temperature treatment step, and can be produced in a so-called roll-to-roll system using a sheet-shaped substrate. Accordingly, cost reduction is expected. However, further improvements in power generation efficiency and durability have been desired for practical use of the organic solar cell. In particular, the development of a perovskite solar cell including a crystal having a perovskite structure as a photoelectric conversion layer toward its practical use has been advanced because the cell is excellent in photoelectric conversion characteristic. For example, in Patent Literature 1, there is a description of a technology including improving photoelectric conversion efficiency by forming, between a hole-transporting layer and a perovskite, a layer containing a phthalocyanine compound. In Patent Literature 2, there is a description of a technology including forming, between a hole-transporting layer and a perovskite, a layer containing a phthalocyanine compound and an aromatic ring compound having a hydroxy group so that conversion efficiency can be maintained over a long time period.

### [Citation List]

### [Patent Literature]

PTL 1: Japanese Patent Laid-Open No. 2022-168820
PTL 2: Japanese Patent Laid-Open No. 2024-60579

### [Summary of Invention]

### [Technical Problem]

According to investigations made by the inventors of the present invention, it has been found that there is still room for improvement in the dispersion stability of the composition described in each of Patent Literature 1 and Patent Literature 2, and the photoelectric conversion efficiency of a photoelectric conversion element using the composition.

Accordingly, the present invention is directed to providing such a composition that the dispersion stability of the composition and the photoelectric conversion efficiency of a photoelectric conversion element using the composition are improved.

### [Solution to Problem]

The above-mentioned provision is achieved by the present invention described below. That is, the present invention is directed to a composition including: a pigment that is a cyclic conjugated compound in which a plurality of pyrrole rings are bonded by conjugated bonds; a dispersant; a resin; and a solvent,
wherein the dispersant is a calixarene compound represented by the following formula [A], and
wherein a mass of the dispersant in the composition is 0.20 times or more with respect to a mass of the resin in the composition: 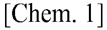 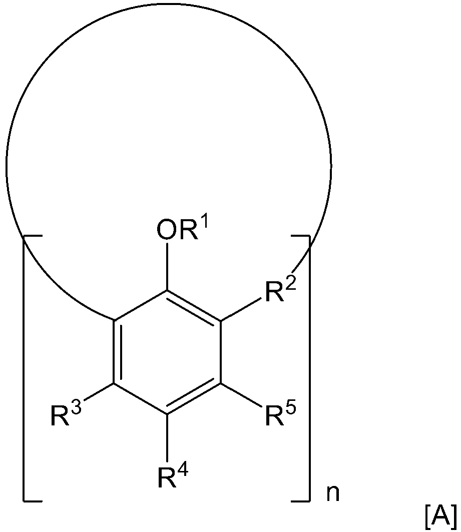 in the formula [A], R¹ to R⁵ are as follows each independently in each repeating unit and each independently for "n" repeating units: R¹ represents a hydrogen atom or an alkyl group; R² represents a substituted or unsubstituted alkylene group; and R³ to R⁵ each represent a hydrogen atom, a substituted or unsubstituted alkyl group, a substituted or unsubstituted -Y-Ar group, a substituted or unsubstituted aromatic hydrocarbon group, or a substituted or unsubstituted heterocyclic group, and at least one of R³ to R⁵ represents a substituted or unsubstituted -Y-Ar group, where -Y- of the -Y-Ar group represents -CH=N-, -CH=CH-, or -N=N-, and Ar represents a substituted or unsubstituted aromatic hydrocarbon group, or a substituted or unsubstituted heterocyclic group, and "n" represents an integer of 3 to 20.

### [Advantageous Effects of Invention]

According to the present invention, the dispersion stability of the composition is improved, and the photoelectric conversion efficiency of the photoelectric conversion element using the composition as a charge-transporting layer coating liquid can be improved.

### [Brief Description of Drawings]

[Fig. 1]

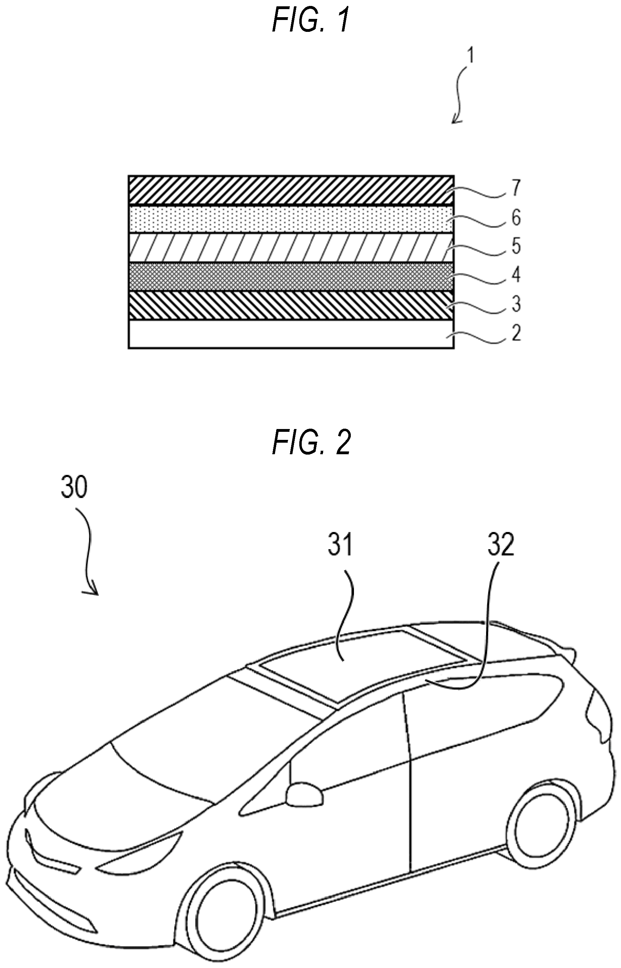

   Fig. 1 is a schematic sectional view in a thickness direction of a photoelectric conversion element of the present invention.
[Fig. 2]
   Fig. 2 is a perspective view for schematically illustrating an example of a moving body including the photoelectric conversion element of the present invention.
[Fig. 3]

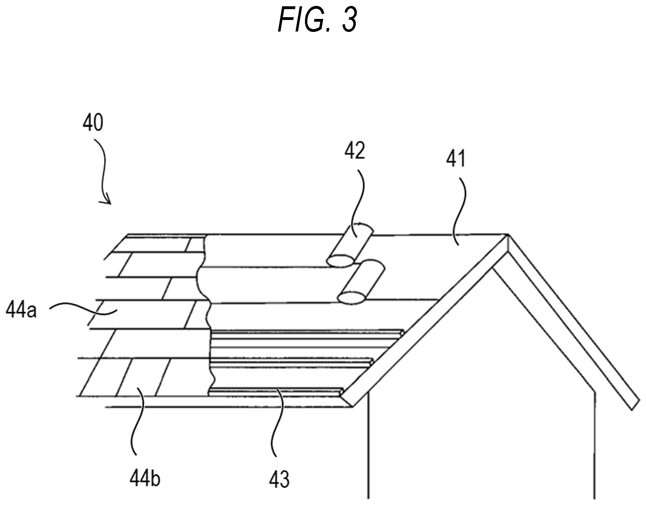

   Fig. 3 is a perspective view for schematically illustrating an example of a building material including the photoelectric conversion element of the present invention.

### [Description of Embodiments]

A composition of the present invention is a composition including: a pigment that is a cyclic conjugated compound in which a plurality of pyrrole rings are bonded by conjugated bonds; a dispersant; a resin; and a solvent,
wherein the dispersant is a calixarene compound represented by the following formula [A], and
wherein a mass of the dispersant in the composition is 0.20 times or more with respect to a mass of the resin in the composition: 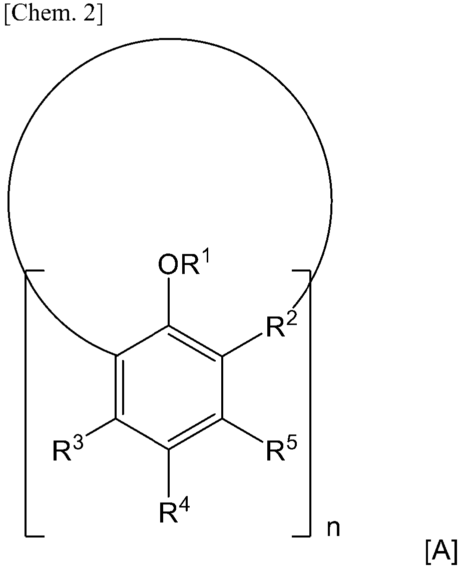 in the formula [A], R¹ to R⁵ are as follows each independently in each repeating unit and each independently for "n" repeating units: R¹ represents a hydrogen atom or an alkyl group; R² represents a substituted or unsubstituted alkylene group; and R³ to R⁵ each represent a hydrogen atom, a substituted or unsubstituted alkyl group, a substituted or unsubstituted -Y-Ar group, a substituted or unsubstituted aromatic hydrocarbon group, or a substituted or unsubstituted heterocyclic group, and at least one of R³ to R⁵ represents a substituted or unsubstituted -Y-Ar group, where -Y- of the -Y-Ar group represents -CH=N-, -CH=CH-, or -N=N-, and Ar represents a substituted or unsubstituted aromatic hydrocarbon group, or a substituted or unsubstituted heterocyclic group, and "n" represents an integer of 3 to 20.

As a result of investigations, the inventors of the present invention have found that the dispersion stability of the above-mentioned composition and the photoelectric conversion efficiency of a photoelectric conversion element using the composition as a charge-transporting layer coating liquid can be improved. The details of the reason why the dispersion stability of the composition and the photoelectric conversion efficiency of the photoelectric conversion element using the composition as the charge-transporting layer coating liquid can be improved in the present invention are not clear, but the mechanism of the improvement is conceived to be as described below.

The term "dispersion stability" as used herein specifically means that the particle diameter of the pigment particle of the composition after its dispersion is 1.0×10¹ to 5.0×10² nm and the particle diameter lasts for a long period, that is, at least 3 months or more.

The particle diameter of the pigment particle of the composition may be measured with Zetasizer Nano ZS (manufactured by Malvern Panalytical Ltd.). The particle diameter may be measured with the apparatus by a dynamic light scattering method. First, the composition is diluted to prepare a dilution so that the solid-liquid ratio of a particle to be measured becomes 0.10 mass% (+/-0.02 mass%). The dilution is collected in a quartz cell and the cell is loaded into a measurement section. The same liquid as a solvent for forming the charge-transporting layer coating liquid is prepared and used as a diluting liquid. For measurement conditions, the refractive index and viscosity of a dispersion solvent at 20°C are input with control software Zetasizer software 6.30, and measurement is performed under the condition that the liquid is 20°C, followed by the determination of a Z-average particle diameter. In the case of a mixed solvent, the weight-average value of dispersion media to be mixed is adopted.

According to the prior art literature, submicron unevenness occurs on a surface when a photoelectric conversion layer contains a crystal having a perovskite structure. It has been presumed that interfacial joining with an electrode is stabilized by filling a recess of the unevenness with a pigment particle formed of a phthalocyanine compound, which is a kind of cyclic compound in which a plurality of pyrrole rings are bonded by conjugated bonds, and hence high photoelectric conversion efficiency can be obtained.

However, it has been found that the foregoing is insufficient, and there is still room for improvement in each of the dispersion stability of the composition and the photoelectric conversion efficiency of the photoelectric conversion element using the composition as the charge-transporting layer coating liquid.

The inventors of the present invention have presumed that the following configuration of the pigment composition contributes to the dispersion stability of the pigment particle of the composition and higher photoelectric conversion efficiency when a photoelectric conversion element is produced by using the composition as the charge-transporting layer coating liquid: a calixarene compound represented by the following formula [A] serving as a dispersant and a resin are added in addition to a solvent and a cyclic conjugated compound in which a plurality of pyrrole rings are bonded by conjugated bonds; and the mass of the dispersant in the composition is 0.20 times or more with respect to the mass of the resin in the composition.

With regard to the dispersion stability, the dispersion stability is reinforced when the cyclic compound in which the plurality of pyrrole rings are bonded by conjugated bonds, the calixarene compound represented by the formula [A], the resin, and the solvent interact with each other. This is presumed to be because a hydrophobic portion (R³, R⁴, and R⁵ sides of the formula [A]) of the calixarene compound having a cup-shaped structure interacts with the cyclic compound in which the plurality of pyrrole rings are bonded by conjugated bonds, and a hydrophilic side (R¹ side of the formula [A]) thereof interacts with the resin and the solvent. Accordingly, it is conceived that the dispersion stability of the pigment particle is poor when any one of the dispersant or the resin is absent.

Further, when the mass of the dispersant in the composition is less than 0.20 times with respect to the mass of the resin in the composition, the resin interacts with the cyclic compound in which the pyrrole rings are bonded by conjugated bonds more than necessary while it is originally desired that the cyclic compound in which the pyrrole rings are bonded by conjugated bonds and the calixarene compound represented by the formula [A] preferentially interact with each other. As a result, as compared to the case of 0.20 times or more, uniform dispersion of the pigment particle is difficult to proceed, cohesiveness increases, and the dispersion stability deteriorates.

With regard to efficiency when the photoelectric conversion element is formed, as a result of the deterioration of the dispersion stability, it is estimated that when the coating liquid is applied onto the perovskite, the ratio of a portion of the perovskite that cannot be filled with a particle of the cyclic compound in which the plurality of pyrrole rings are bonded by conjugated bonds increases, the interfacial joining becomes more unstable, and as a result, the photoelectric conversion efficiency is lowered.

Through the mechanism described above, it is conceived that when the respective configurations synergistically affect each other, the dispersion stability of the pigment particle composition is improved, and the perovskite can be densely filled, and hence the interfacial joining with the electrode is stabilized to improve the photoelectric conversion efficiency.

In the composition of the present invention, the solvent is a solvent that does not break a perovskite crystal. As a method of selecting the solvent that does not break a perovskite crystal, it is recognized that the perovskite crystal is not broken, specifically, that the concentration and color of the film have not changed after the solvent has been applied onto a perovskite coating film and then left for 1 hour.

In addition, the solvent is required to be rapidly dried after its application onto the perovskite, and hence preferably has a vapor pressure (20°C) of 0.06 kPa or more. When the solvent is rapidly dried, a defect such as particles aggregating and being unable to sufficiently cover the perovskite can be reduced. When the above-mentioned condition is satisfied under a state in which two or more kinds of solvents are mixed, the solvents may be used as a mixed solvent. The vapor pressure in the case of two or more kinds of solvents is determined as a value obtained by adding up products of the vapor pressures of the respective solvents and their weight ratios in the mixed solvent. Specific examples of the solvent are not limited as long as the following two conditions are satisfied: the above-mentioned perovskite crystal is not broken; and the vapor pressure falls within the above-mentioned range, and include hydrocarbon-based, ester-based, ketone-based, ether-based, aromatic compound-based, and alcohol-based solvents. Of those, an alcohol-based solvent is preferred as a main solvent from the viewpoints of the satisfaction of the above-mentioned conditions and the dispersion stability.

In the composition of the present invention, the cyclic compound in which the plurality of pyrrole rings are bonded by conjugated bonds, the cyclic compound serving as a pigment, is dispersed in a coating liquid and is present in a particulate form. When the cyclic compound is formed into a film in a particle state, high crystallinity is maintained and an inherent high charge-transporting ability can be exhibited.

Specific examples of the cyclic compound in which the plurality of pyrrole rings are bonded by conjugated bonds in the present invention include: porphyrin derivatives, such as tetraphenylporphyrin, diphenylporphyrin, tetrapyridylporphyrin, copper porphyrin, copper tetraphenylporphyrin, copper octaethylporphyrin, cobalt tetraphenylporphyrin, octaethylporphyrin, chlorophenylporphyrin, methoxyphenylporphyrin, methylphenylporphyrin, zinc porphyrin, magnesium porphyrin, octabutoxyporphyrin, manganese chloroporphyrin, metal-free tetraazaporphyrin, copper tetraazaporphyrin, zinc tetraazaporphyrin, nickel tetraazaporphyrin, titanyl tetraazaporphyrin, tetraphenyltetraazaporphyrin, and octaphenyltetraazaporphyrin; phthalocyanine derivatives, such as hydroxygallium phthalocyanine, chlorogallium phthalocyanine, copper phthalocyanine, zinc phthalocyanine, phthalocyanine, cobalt phthalocyanine, titanyl phthalocyanine, dichlorotin phthalocyanine, magnesium phthalocyanine, tin phthalocyanine, lead phthalocyanine, iron phthalocyanine, vanadyl phthalocyanine, chloroaluminum phthalocyanine, nickel phthalocyanine, dichlorosilicon phthalocyanine, indium chlorophthalocyanine, manganese phthalocyanine, chloroiron phthalocyanine, and platinum phthalocyanine; and naphthalocyanine derivatives, such as naphthalocyanine, magnesium naphthalocyanine, copper naphthalocyanine, cobalt naphthalocyanine, vanadyl naphthalocyanine, tin naphthalocyanine, and dichlorotin naphthalocyanine.

In the present invention, a porphyrin compound and a phthalocyanine compound are preferred, and a phthalocyanine compound is more preferred from the viewpoint of the expansion of a π-electron cloud serving as the starting point of an interaction with perovskite. The central element of the phthalocyanine compound is preferably metal-free, or at least one of gallium, aluminum, titanium, iron, or silicon. Phthalocyanine, gallium phthalocyanine, titanyl phthalocyanine, aluminum phthalocyanine, iron phthalocyanine, and silicon phthalocyanine are each particularly preferred as the phthalocyanine compound. Of those, gallium phthalocyanine is more preferred, and a hydroxygallium phthalocyanine compound is particularly preferred. In addition, when the X-ray diffraction spectrum of a film of the composition is measured, the ratio of the intensity of a peak in the range of a Bragg angle 2θ of 7.6 to 8.6° with respect to the total peak intensity of 0.0 to 30.0° is preferably 0.02 or more from the viewpoint of an electronic interaction. In the present invention, the chemical structure of, for example, the cyclic conjugated compound in which the plurality of pyrrole rings are bonded by conjugated bonds may be identified by, for example, a nuclear magnetic resonance method (NMR).

In the composition of the present invention, the dispersant is a calixarene compound represented by the following formula [A]: 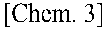 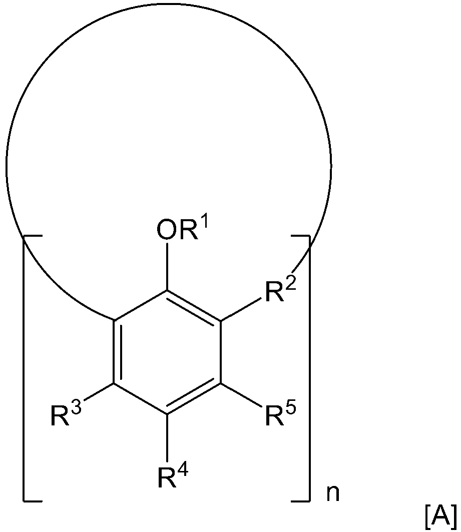 in the formula [A], R¹ to R⁵ are as follows each independently in each repeating unit and each independently for "n" repeating units: R¹ represents a hydrogen atom or an alkyl group; R² represents a substituted or unsubstituted alkylene group; and R³ to R⁵ each represent a hydrogen atom, a substituted or unsubstituted alkyl group, a substituted or unsubstituted -Y-Ar group, a substituted or unsubstituted aromatic hydrocarbon group, or a substituted or unsubstituted heterocyclic group, and at least one of R³ to R⁵ represents a substituted or unsubstituted -Y-Ar group, where -Y- of the -Y-Ar group represents -CH=N-, -CH=CH-, or -N=N-, and Ar represents a substituted or unsubstituted aromatic hydrocarbon group, or a substituted or unsubstituted heterocyclic group, and "n" represents an integer of 3 to 20.

Examples of the alkyl group represented by each of R¹ to R⁵ include a methyl group, an ethyl group, a propyl group, and a butyl group.

Examples of the aromatic hydrocarbon group represented by each of R³ to R⁵ include benzene, naphthalene, fluorene, phenanthrene, anthracene, fluoranthene, and pyrene.

In addition, examples of the heterocyclic group represented by each of R³ to R⁵ include furan, thiophene, pyridine, indole, benzothiazole, carbazole, benzocarbazole, acridone, dibenzothiophene, benzoxazole, benzotriazole, oxathiazole, thiazole, phenazine, cinnoline, and benzocinnoline.

In addition, examples of a substituent that the alkyl group, the phenylazo group, the aromatic hydrocarbon group, or the heterocyclic group represented by each of R² to R⁵ may have include: alkyl groups, such as a methyl group, an ethyl group, a propyl group, and a butyl group; alkoxy groups, such as a methoxy group and an ethoxy group; dialkylamino groups, such as a dimethylamino group and a diethylamino group; alkoxycarbonyl groups, such as a methoxycarbonyl group and an ethoxycarbonyl group; halogen atoms, such as a fluorine atom, a chlorine atom, and a bromine atom; a hydroxy group; a nitro group; a cyano group; and a halomethyl group.

In the aromatic ring compound having a calixarene structure represented by the formula [A], "n" preferably represents 4 to 8 and its molecular weight is preferably 10,000 or less from the viewpoint of its molecular size in order to easily improve dispersion stability.

In addition, in the present invention, R¹ preferably represents a hydrogen atom, a methyl group, an ethyl group, or a propyl group each independently for "n" repeating units. In addition, R² preferably represents a methylene group, an ethylene group, or a trimethylene group each independently for "n" repeating units. It is preferred that R³ and R⁵ each represent a hydrogen atom, and R⁴ represent a nitrophenylazo group or a dinitrophenylazo group each independently for "n" repeating units.

Of those, specific examples of the calixarene compound to be particularly preferably used in the present invention are given below. In the present invention, the composition preferably includes, as the dispersant, at least one selected from the group consisting of: a compound represented by the following formula [C-1]; a compound represented by the following formula [C-2]; a compound represented by the following formula [C-3]; and a compound represented by the following formula [C-4], and more preferably includes all the four compounds (mixed product).

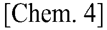

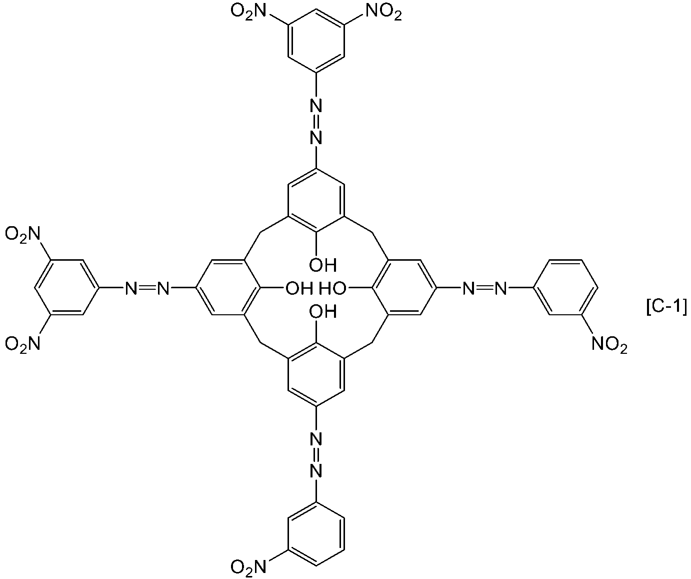

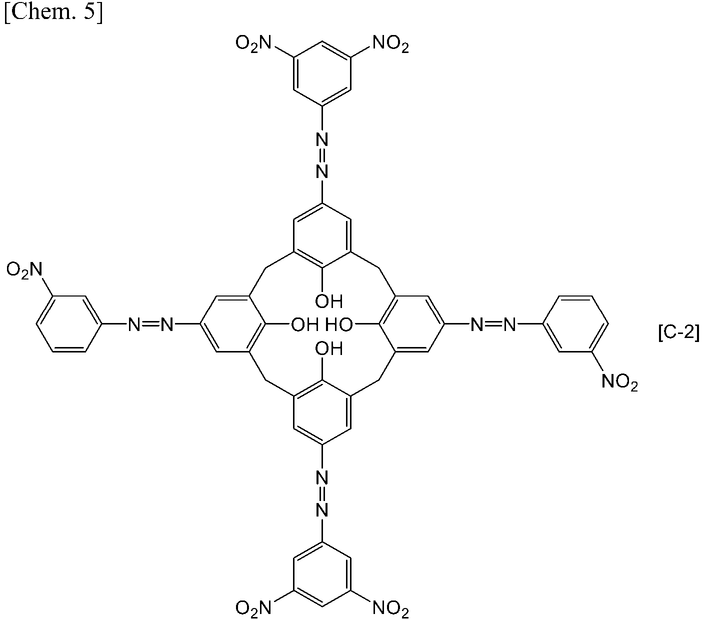

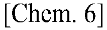

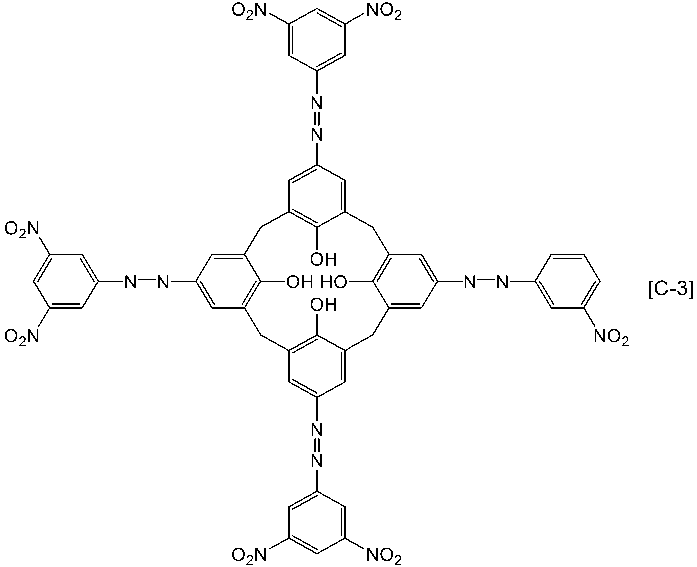

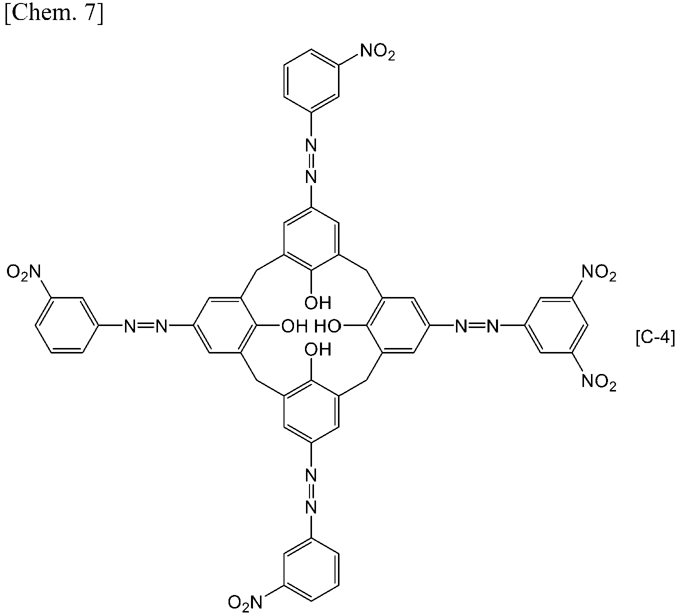

In the present invention, the chemical structure of the aromatic ring compound having a calixarene structure represented by the formula [A] or the like may be identified by, for example, a nuclear magnetic resonance method (NMR).

The mass of the dispersant in the composition of the present invention is preferably 0.01 to 0.50 times with respect to the mass of the pigment particle, which is a cyclic compound in which a plurality of pyrrole rings are bonded by conjugated bonds, in the composition from the viewpoint of the dispersion stability of the pigment particle.

In the composition of the present invention, specific examples of the resin include a polyacetal resin, an acrylic resin, a polyarylate resin, a polycarbonate resin, a polyvinyl acetate resin, a polyester resin, a polyamide resin, a polyurethane resin, and a polystyrene resin.

The resin is preferably a resin having a Lewis basic functional group. When the Lewis basic functional group electronically acts on the pigment particle, which is a cyclic compound in which a plurality of pyrrole rings are bonded by conjugated bonds, and the dispersant, which is a calixarene compound, an interaction between the pigment particle and the resin increases, the resin easily intervenes between the particles, and an effect of steric barrier stabilization by the resin is easily obtained.

Specific examples of the Lewis basic functional group include a hydroxy group, a halogen, a sulfo group, an amino group, a carbonyl group, an ester bond, an ether bond, a carboxyl group, an aldehyde group, a methoxy group, an amide group, a sulfide group, a cyano group, a thienyl group, a pyridyl group, furan, pyrazole, imidazole, oxazole, and thiazole. Of those, a hydroxy group, a carbonyl group, an ether bond, an ester bond, a pyridyl group, and a thienyl group are preferred from the viewpoint of an electronic interaction. In particular, the resin more preferably has at least two of those functional groups from the viewpoint of the ease of an interaction. One kind of resin may have different functional groups, or two kinds of resins having different functional groups may be mixed. The Lewis basic functional group is preferably included in the repeating structure of the resin.

Specific examples of the resin having a Lewis basic functional group to be preferably used in the present invention include polyvinyl butyral, poly(4-vinylpyridine), poly(vinyl chloride), poly(vinylidene fluoride), polyacrylonitrile, poly(vinylidene fluoride-co-hexafluoropropylene), poly(acrylonitrile-co-butadiene), poly(styrene-co-acrylonitrile), polychloroprene, poly(4-chlorostyrene), polymethyl methacrylate, polyvinyl acetate, polyethyleneimine, polyvinyl alcohol, polyacrylic acid, poly(sodium 4-styrenesulfonate), poly(allylamine hydrochloride), sodium polyacrylate, poly(4-styrenesulfonic acid), poly(N-isopropylacrylamide), poly(2-ethyl-2-oxazoline), poly(ethylene-alt-maleic anhydride), poly(2-acrylamido-2-methyl-1-propanesulfonic acid), poly(vinyl sulfate) potassium salt, polyanetholesulfonic acid sodium salt, poly(2-(dimethylamino)ethyl methacrylate) methyl chloride quaternary salt, poly(methyl vinyl ether), poly(2-propylacrylic acid), polyvinylpyrrolidone, polypropylene glycol, poly(propylene carbonate), polyvinyl acetate, poly(tetrahydrofuran), nylon-6, poly(ethylene-co-vinyl acetate), poly(propylene glycol) bis(2-aminopropyl ether), poly(bisphenol A carbonate), poly(1,4-butylene adipate), poly(4-vinylphenol), poly(propylene glycol) monobutyl ether, poly(glycidyl methacrylate), poly(butyl acrylate), poly(ethylene succinate), poly(propylene glycol) methacrylate, nylon 11, nylon 12, poly(2-ethylhexyl acrylate), poly(propylene glycol) bis(2-aminopropyl ether), polyetherimide, poly(vinyl formal), poly(vinyl methyl ketone), poly(3-hexylthiophene-2,5-diyl), polyaniline, and a composite of poly(3,4-ethylenedioxythiophene) and polystyrenesulfonic acid (PEDOT:PSS). Of those, polyvinyl butyral, polymethyl methacrylate, poly(3-hexylthiophene-2,5-diyl), polyvinyl acetate, polyvinyl alcohol, polyacrylic acid, poly(2-propylacrylic acid), poly(butyl acrylate), and poly(4-vinylpyridine) are more preferred, and a polyvinyl acetal resin and a polyvinyl butyral resin are particularly preferred from the viewpoint of an electronic interaction. Each of those resin easily interacts with the pigment particle, which is a cyclic compound in which a plurality of pyrrole rings are bonded by conjugated bonds.

It is more preferred that the glass transition temperature (Tg) of the resin be 95°C or less. When the glass transition temperature falls within the above-mentioned range, the resin is easily brought into close contact with the pigment particle, which is a charge-transporting particle, and an improvement in coverage by an interaction becomes more effective. The glass transition temperature may be determined with a differential scanning calorimeter (DSC).

The molecular weight of the resin preferably falls within the range of 1,000 to 1,000,000 in terms of weight-average molecular weight from the viewpoints of dispersibility and film formability.

The mass of the pigment particle, which is a cyclic compound in which a plurality of pyrrole rings are bonded by conjugated bonds, in the composition of the present invention is preferably 5 to 20 times with respect to the mass of the resin in the composition because the resin easily interacts with the pigment particle and the coverage is easily improved. When the mass of the pigment particle is more than 20 times, the improvement in coverage by the interaction of the resin tends to be insufficient. When the mass of the pigment particle is less than 5 times, charge transfer from the photoelectric conversion layer to the particle becomes insufficient, and hence it becomes difficult to improve the photoelectric conversion efficiency.

A method of dispersing the composition of the present invention is, for example, a method including using a paint shaker, a sand mill, a ball mill, or a liquid collision type high-speed disperser. Of those, a sand mill performs dispersion by the rotation of a disc rotating in a mill and shearing force by media such as glass beads serving as grinding media. Dispersion stability may be changed by dispersion conditions at that time, such as a dispersion time, the amount of beads, the number of rotations of the disc, and the timing of the addition of each material.

In the preparation of the composition of the present invention, particularly with regard to the timing of the addition of the resin in a process for producing the composition, it is required to first perform dispersion to some extent with a pigment, a dispersant, and a solvent to provide a dispersion liquid (pre-dispersion liquid), and then add the resin to the dispersion liquid later to perform dispersion again, rather than adding the resin together with the pigment, the dispersant, and the solvent from the start of dispersion. Thus, the dispersion stability of the pigment is improved.

When the dispersion stability is improved by, for example, devising the production method, differences in dispersion stability that cannot be completely determined by a particle diameter measured with the Zetasizer Nano ZS (manufactured by Malvern Panalytical Ltd.) due to influences of aggregation or the like may be recognized by an imaging method using a scanning electron microscope (SEM) image or the measurement of a specific surface area coefficient by pulsed NMR as required.

In addition, the measurement of the X-ray diffraction spectrum of the composition and the measurement of the content of the cyclic compound or the like in which a plurality of pyrrole rings are bonded by conjugated bonds therein are described. The analysis of the content of the compound amount and the X-ray diffraction measurement were performed by the following method by using a dried film of the composition or by exposing a surface of a film of the composition after removing an upper layer of the charge-transporting layer of a photoelectric conversion element using the composition as the charge-transporting layer coating liquid with an organic solvent such as chloroform.

### [Analysis of Compound Amount]

The surface of the film of the composition was wiped with a cotton swab or the like to which a solvent was applied, dissolved in deuterated sulfuric acid, and subjected to ¹H-NMR measurement (apparatus: AVANCE III-500, manufactured by Bruker). In addition, the wiped component was subjected to GPC, MALDI-TOF-MS, IR, gas chromatography, and elemental analysis, such as XPS or EDX. Those mass and structural analyses were performed to determine the presence of the compound and an important ratio with the resin or the dispersant.

In addition, the thickness of the film was determined with a cross-sectional SEM (apparatus: SmartSEM manufactured by Carl Zeiss Co., Ltd.) after the cutting of the photoelectric conversion element and the fixing of the sample to a tilted sample stage.

### ·MALDI-TOF-MS Analysis

The analysis was performed under the following conditions, and a molecular weight was determined from the resultant peak top value.

Measurement instrument used: matrix-assisted laser desorption/ionization time-of-flight mass spectrometer (MALDI-TOF MS) ultraflex manufactured by Bruker Daltonics Co., Ltd.
Acceleration voltage: 20 kV
Mode: Reflector
Molecular weight standard product: Fullerene C60

### [X-ray Diffraction Measurement]

The X-ray diffraction spectrum of the film of the composition was measured, and the ratio of the intensity of a peak in the range of a Bragg angle 2θ of 7.6 to 8.6° with respect to the total peak intensity of 0.0 to 30.0° was determined.
Measurement instrument used: X-ray diffractometer RINT-TTRII manufactured by Rigaku Corporation
X-ray tube: Cu
X-ray wavelength: Kα1
Tube voltage: 50 kV
Tube current: 300 mA
Scan method: 2θ-θ scan
Scan speed: 0.4°/min
Sampling interval: 0.005°
Start angle 2θ: 3.0°
Stop angle 2θ: 30.0°
Goniometer: Rotor horizontal goniometer (TTR-2)
Filter: none
Detector: scintillation counter
Incident monochromator: used
Slit: variable slit (parallel beam method)
Counter monochromator: not used
Divergence slit: open
Divergence longitudinal limiting slit: 10.00 mm
Scattering slit: open
Receiving slit: open

The present invention is described in detail below by way of preferred embodiments. The present invention is not limited to the following embodiments, and the following embodiments, which are appropriately changed, modified, and the like based on the ordinary knowledge of a person skilled in the art without departing from the gist of the present invention, are also encompassed within the scope of the present invention.

The term "layer" as used herein means not only a layer having a clear boundary or a layer having a flat thin film shape but also a layer having a concentration gradient in which the concentration of a constituent element gradually changes, or a layer that may form a complicatedly intricate structure together with another layer. The elemental analysis of the layer may be performed by, for example, performing the TOF-SIMS/FE-TEM/EDS line analysis measurement of a cross section of the photoelectric conversion element and determining the element distribution of a specific element. The analysis of each layer may be performed by peeling and removing a layer from a completed photoelectric conversion element to expose the layer to be analyzed. In addition, the analysis of the compound in the charge-transporting layer coating liquid may be performed by mass and structural analysis through ¹H-NMR, GPC, MALDI-TOF-MS, IR, gas chromatography, liquid chromatography, or elemental analysis, such as XPS or EDX.

Fig. 1 is a sectional view for schematically illustrating the configuration of an embodiment of a photoelectric conversion element using the composition of the present invention as a charge-transporting layer coating liquid. A photoelectric conversion element 1 includes a substrate 2, and a second electrode 3, an electron-transporting layer 4, a photoelectric conversion layer 5, a charge-transporting layer 6, and a first electrode 7 arranged thereon. One of the first electrode 7 and the second electrode 3 is an anode, and the other is a cathode. A current can be extracted by connecting the first electrode 7 and the second electrode 3 with an external circuit.

The photoelectric conversion layer 5 is excited by light that has entered the layer through the substrate 2, the second electrode 3, and the electron-transporting layer 4, or the first electrode 7 and the charge-transporting layer 6 to generate an electron or a hole. That is, the photoelectric conversion layer 5 generates a current between the first electrode 7 and the second electrode 3. The electron-transporting layer 4 is a layer arranged between the photoelectric conversion layer 5, and the two electrodes (the second electrode 3 and the first electrode 7), and may not be formed in some cases. A form in which the plurality of electron-transporting layers 4 and photoelectric conversion layers 5 are laminated may be adopted. Such form may also be referred to as "tandem structure." The respective members are described below.

### [Photoelectric Conversion Element]

The photoelectric conversion element of the present invention is characterized by including: the first electrode; the second electrode; the photoelectric conversion layer arranged between the first electrode and the second electrode, the photoelectric conversion layer containing the crystal having a perovskite structure; and the charge-transporting layer between the photoelectric conversion layer and the first electrode. In addition, in order to improve the photoelectric conversion efficiency, a tandem type in which the photoelectric conversion elements are laminated may be adopted. The kind of the photoelectric conversion element to be laminated is not limited, and for example, a silicon solar cell or a CIGS solar cell may be adopted in addition to a perovskite solar cell using a perovskite crystal in its photoelectric conversion layer.

A method of forming each of the layers of the photoelectric conversion element of the present invention is, for example, a coating method or a vapor deposition method. Examples of the coating method include dip coating, spin coating, spray coating, ink jet coating, meniscus coating, screen coating, roll coating, die coating, blade coating, curtain coating, and wire bar coating. The coating method is a method including preparing a coating liquid for each layer to be described later, applying the liquid in the desired order of layers, and drying the liquid. A desired method may be selected as such forming method in accordance with each layer.

The respective layers are described below.

### [Substrate]

The photoelectric conversion element 1 of the present invention may include the substrate 2, and examples thereof include a transparent glass substrate made of soda-lime glass or alkali-free glass, a ceramic substrate, and a transparent plastic substrate. When light is taken in from the first electrode 7 side, an opaque material may be used as the substrate 2, and when light is taken in from the second electrode 3 side, the substrate 2 is formed of a transparent material.

### [Electrode]

A material for the first electrode 7 or the second electrode 3 is not particularly limited, and a material that has hitherto been known may be used. Examples thereof include: metals, such as gold, silver, titanium, and copper; sodium; a sodium-potassium alloy; lithium; magnesium; carbon; aluminum; a magnesium-silver mixture; a magnesium-indium mixture; an aluminum-lithium alloy; an Al/Al₂O₃ mixture; and an Al/LiF mixture. Examples of a transparent electrode material include: conductive transparent materials, such as CuI, indium tin oxide (ITO), SnO₂, aluminum zinc oxide (AZO), indium zinc oxide (IZO), gallium zinc oxide (GZO), fluorine-doped tin oxide (FTO), and antimony-doped tin oxide (ATO); and conductive transparent polymers. Those materials may be used alone or in combination thereof. At least one electrode of the first electrode 7 or the second electrode 3 on a light incident side is a transparent electrode, and the other may be a transparent electrode or may also serve as a reflective layer formed of a light reflective material, or may be a transparent electrode including a reflective layer on a side opposite to the light incident side. When the first electrode 7 is on the light incident side, the second electrode 3 and the substrate 2 may be a transparent electrode and a reflective layer, respectively. The electrode may be a patterned electrode.

### [Photoelectric Conversion Layer]

The photoelectric conversion layer 5 contains the crystal having a perovskite structure. The crystal having a perovskite structure to be used in the present invention is preferably represented by the following general formula [1].

ABX₃ [1]

In the general formula [1], A represents a monovalent cation of an organic molecule or a metal atom, B represents a divalent metal cation, and X represents a monovalent halide anion.

A in the general formula [1] preferably represents CₚNₘHₙ ("p", "m", and "n" each represent a positive integer) in the case of, for example, the organic molecule. Specific examples thereof include methylammonium and formamidinium.

In addition, the metal atom is not particularly limited, and lithium, cesium, sodium, potassium, and rubidium are preferred. Those organic molecules or metal atoms may be used alone or in combination thereof.

When the cation A to be included is too large to fit in a crystal having a three-dimensional perovskite structure, a crystal having a two-dimensional perovskite structure, a crystal having a 2.5-dimensional perovskite structure with properties of both the two-dimensional and three-dimensional perovskite structures, a two-layer crystal having three-dimensional and two-dimensional perovskite structures, or a crystal having a mixed three-dimensional/two-dimensional perovskite structure is formed, and any of the structures functions as the photoelectric conversion layer. The two-layer crystal having three-dimensional and two-dimensional perovskite structures refers to a crystal in which the crystals having three-dimensional and two-dimensional perovskite structures are laminated as independent and separate layers. The crystal having a mixed three-dimensional/two-dimensional perovskite structure refers to a crystal having a structure in which both the regions or domains of crystals having two-dimensional or 2.5-dimensional layered and three-dimensional perovskite structures are mixed.

It is preferred that the crystal having a two-dimensional perovskite or 2.5-dimensional perovskite structure be represented by each of the following general formulae [2] to [4].

R'₂Aₙ₋₁BₙX₃ₙ₊₁ [2]

R"Aₙ₋₁BₙX₃ₙ₊₁ [3]

R‴AₙBₙX₃ₙ₊₁ [4]

The general formula [2], the general formula [3], and the general formula [4] form perovskite structures of a Ruddlesden-Popper (RP) type, a Dion-Jacobson (DJ) type, and an Alternating cations in the interlayer (ACI) type, respectively.

R', R", and R‴ in the general formulae [2] to [4] each preferably represents CₚNₘHₙ ("p", "m", and "n" each represent a positive integer) in the case of, for example, the organic molecule. In addition, A may or may not have a substituent. Specifically, ethylammonium, propylammonium, n-butylammonium, n-hexylammonium, n-octylammonium, 1,6-hexadiammonium, iso-butylammonium, 3-(nonafluoro-tert-butyloxy)propylamine, 1,3-propanediammonium, 1,5-pentamethylenediamine, octyldiammonium, 2,2-(ethylenedioxy)bis(ethylammonium), 5-aminovaleric acid, 4-tert-butylammonium, N,N'-dimethylethylene-1,2-diammonium, 2,2,3,3,3-pentafluoropropylammonium, guanidinium, propylammonium, propargylamine, an alkylammonium, cyclohexylmethylammonium, 4-(aminomethyl)piperidinium, piperidinium, pyrrolidinium, cyclohexylammonium, 4-fluorophenethylammonium, 4-fluorophenethylammonium, trifluoromethylbenzylammonium, pentafluorobenzylammonium, pentafluorophenylethylammonium, 4-methoxyphenethylammonium, imidazolium, pyridinium, 3-thiophenemethylammonium, 2-thiopheneethylammonium, 2-thiopheneformamidinium, 2-thiophenemethylammonium, 1-naphthylmethylammonium, 2-naphthylmethylammonium, phenethylammonium, phenylammonium, benzylammonium, 2,5-thiophenedimethylammonium, phenylpropylammonium, 1,4-phenylenedimethanamine, 3-phenyl-2-propen-1-ammonium, phenylbutylammonium, 4-tert-butylbenzylammonium, 3-(aminomethyl)piperidinium, and 4-(aminomethyl)piperidinium are preferred.

B in each of the general formulae [1] to [4] represents a metal atom, and examples thereof include lead, tin, bismuth, zinc, titanium, antimony, nickel, iron, cobalt, silver, copper, gallium, germanium, magnesium, calcium, indium, aluminum, manganese, chromium, molybdenum, and europium. Of those, lead, tin, and bismuth are preferred from the viewpoint of the overlap of electron orbits. Those metal atoms may be used alone or in combination thereof.

X in each of the general formulae [1] to [4] represents a halogen atom, and examples thereof include chlorine, bromine, and iodine. Those halogen atoms may be used alone or in combination thereof. Of those, a halogen atom is preferred because, when the halogen atom is incorporated into the structure, the above-mentioned crystal having a perovskite structure easily becomes soluble in an organic solvent, and hence the application to an inexpensive printing method or the like is enabled. Further, iodine is more preferred because the energy bandgap of the crystal having a perovskite structure narrows.

Specifically, as three-dimensional perovskite, two-dimensional perovskite, and mixed three-dimensional/two-dimensional perovskite, MAPbI₃, FAPbCl₃, FAPbI₃, MAPbIₓBr₃₋ₓ, MAPbIₓCl₃₋ₓ, Cs_{0.05}(MA_{0.17}FA_{0.83})_{0.95}Pb(I_{0.83}Br_{0.17})₃, {Csₓ₁(FAₓ₂MA₁₋ₓ₂)₁₋ₓ₁}ₓ₃Pb(Iₓ₄Br₁₋ₓ₄)ₓ₅, Cs_{0.05}FA_{0.88}MA_{0.07}PbI_{2.56}Br_{0.44}, (FAPbI₃)_{0.95}(MAPbBr₃)_{0.05}, (FAPbI₃)_{0.85}(MAPbBr₃)_{0.15}, CsPbI₃, CsPbBr₃, Csₓ(MA)₁₋ₓPbI₃, Csₓ(FA)₁₋ₓPbI₃, MAₓ(FA)₁₋ₓPbI₃, MA_{0.17}FA_{0.83}Pb(I_{0.83}Br_{0.17})₃, Cs0.15FA0.85PbI2.55Br0.45, Cs0.05FA0.88MA0.07PbI2.56Br0.44, Cs0.15FA0.85PbI2.55Br0.45, (PEA)₂(MA)₂Pb₃I₁₀, (PTA)₂(MA)₄Pb₅I₁₆, (PEA)₂(MA)₄Pb₅I₁₆, (ThMA)₂(MA)₂Pb₃I₁₀, (3BBA)₂(MA)₂Pb₃I₁₀, (ThMA)₂(FA)₄Pb₅I₁₆, (pF-PEA)₂(FA_{0.3}MA_{0.7})₄Pb₅I₁₆, (PDMA)FA₂Pb₃I₁₀, (3AMPY)(MA)₃Pb₄I₁₃, (PDMA)MA₅Pb₆I₁₉, (PDMA)MA₃Pb₄I₁₃, (TTDMA)MA₃Pb₄I₁₃, (TTDMA)MA₄Pb₅I₁₆, (BA_{0.9}PEA_{0.1})₂MA₄Pb₅I₁₆, (BA_{0.9}PEA_{0.1})₂MA₃Pb₄I₁₃, (4FPEA)₂MA₃Pb₄I₁₃, (4FPEA)₂MA₄Pb₅I₁₆, (BA)₂MA₂Pb₃I₁₀, (BA)₂MA₃Pb₄I₁₃, (TEA)₂MA₂Pb₃I₁₀, (BA)₂MA₄Pb₅I₁₆, and (BA)₂MA₃Pb₄I₁₃ are preferred. The A site, B site, or X site of each of the general formulae may be adjusted to be deficient or excessive in accordance with purposes, and the combinations of x1 to x5 may be changed in accordance with purposes. The combinations of x1 to x5 are, for example, as shown in Table 1. Particularly preferred ranges are 0.03≤x1≤0.10, 0.80≤x2≤0.96, 0.95≤x3≤1.05, 0.80≤x4≤0.96, and 2.95≤x5≤3.05. MACl may be incorporated as a material for forming a perovskite crystal.

**[Table 1]**

| x1 | x2 | 1-x2 | x3 | x4 | 1-x4 | x5 |
|---|---|---|---|---|---|---|
| 0.05 | 0.83 | 0.17 | 1.00 | 0.83 | 0.17 | 3.00 |
| 0.05 | 0.83 | 0.17 | 0.99 | 0.83 | 0.17 | 2.99 |
| 0.05 | 0.83 | 0.17 | 0.98 | 0.83 | 0.17 | 2.98 |
| 0.05 | 0.83 | 0.17 | 0.97 | 0.83 | 0.17 | 2.97 |
| 0.05 | 0.83 | 0.17 | 0.96 | 0.83 | 0.17 | 2.96 |
| 0.05 | 0.83 | 0.17 | 1.01 | 0.83 | 0.17 | 3.01 |
| 0.05 | 0.83 | 0.17 | 1.02 | 0.83 | 0.17 | 3.02 |
| 0.05 | 0.83 | 0.17 | 1.03 | 0.83 | 0.17 | 3.03 |
| 0.05 | 0.83 | 0.17 | 1.04 | 0.83 | 0.17 | 3.04 |
| 0.05 | 0.83 | 0.17 | 1.00 | 0.95 | 0.05 | 3.00 |
| 0.05 | 0.83 | 0.17 | 0.97 | 0.95 | 0.05 | 2.97 |
| 0.05 | 0.83 | 0.17 | 0.98 | 0.95 | 0.05 | 2.98 |
| 0.05 | 0.83 | 0.17 | 0.99 | 0.95 | 0.05 | 2.99 |
| 0.05 | 0.83 | 0.17 | 1.01 | 0.95 | 0.05 | 3.01 |
| 0.05 | 0.83 | 0.17 | 1.02 | 0.95 | 0.05 | 3.02 |
| 0.05 | 0.83 | 0.17 | 1.03 | 0.95 | 0.05 | 3.03 |

In the above-mentioned specific examples, "MA" represents methylammonium, "FA" represents formamidinium, "PEA" represents phenethylammonium, "PTA" represents phenyltriethylammonium, "ThMA" represents 2-thiophenemethylammonium, "3BBA" represents 3-bromobenzylammonium, "3AMPY" represents 3-(aminomethyl)pyridine, "PDMA" represents 1,4-phenylenedimethanammonium, "TTDMA" represents thieno[3,2-b]thiophene-2,5-diyldimethanammonium, "4FPEA" represents 4-fluorophenethylammonium, "BA" represents butylammonium, and "TEA" represents 2-thiopheneethylammonium.

The above-mentioned crystal having a perovskite structure preferably has a cubic structure in which the metal atom B, the organic molecules A, and the halogen atom X are arranged on a body-centered position, the respective corners, and a face-centered position, respectively. The details are not clear, but it is assumed that, when such structure is present, the orientation of an octahedron in a crystal lattice can be easily changed, and hence the mobility of an electron in the crystal having a perovskite structure increases, and the photoelectric conversion efficiency of the photoelectric conversion element is improved.

An organic-inorganic perovskite compound to be used in the present invention is preferably a crystalline semiconductor. The term "crystalline semiconductor" means a semiconductor that enables the measurement of an X-ray scattering intensity distribution to detect a scattering peak. When the organic-inorganic perovskite compound is the crystalline semiconductor, the mobility of the electron in the organic-inorganic perovskite compound increases, and the photoelectric conversion efficiency of the photoelectric conversion element is improved.

The thickness of the photoelectric conversion layer according to the present invention is preferably 5 to 2,000 nm. When the thickness is 5 nm or more, light can be sufficiently absorbed, and when the thickness is 2,000 nm or less, the generated charge can be transported to the respective electrodes. A more preferred lower limit is 50 nm or more, a more preferred upper limit is 1,200 nm, a still more preferred lower limit is 100 nm, and a still more preferred upper limit is 1,000 nm.

### [Charge-transporting Layer]

In the photoelectric conversion element of the present invention, the charge-transporting layer is arranged between the photoelectric conversion layer and the first electrode, and the charge-transporting layer contains a charge-transporting substance and a resin, and is arranged on the surface of the photoelectric conversion layer.

The charge-transporting layer may be formed by preparing a coating liquid for a charge-transporting layer, which is the above-mentioned composition, forming a coating film of the coating liquid on the photoelectric conversion layer, and drying the coating film. A method of forming the coating film is, for example, dip coating, spin coating, spray coating, ink jet coating, meniscus coating, screen coating, roll coating, die coating, blade coating, curtain coating, or wire bar coating.

The photoelectric conversion element of the present invention may include a second charge-transporting layer between the first electrode and the charge-transporting layer. When the photoelectric conversion element includes the second charge-transporting layer, the transfer of carriers to an electrode may be facilitated.

The thickness of the charge-transporting layer is preferably 1 to 1,000 nm, more preferably 5 to 500 nm, particularly preferably 10 to 200 nm.

### [Second Charge-transporting Layer]

In the present invention, the photoelectric conversion element 1 may further include the second charge-transporting layer between the charge-transporting layer 6 and the first electrode 7 from the viewpoint of the compatibility of a film of the charge-transporting layer 6.

A material for the second charge-transporting layer is not particularly limited, and examples thereof include a spirofluorene compound, a triphenylamine compound, a chrysene compound, a pyrene compound, a phthalocyanine compound, a carbazole compound, a fluorene compound, a phenylcyclohexane compound, a benzidine compound, a phenoxazine compound, a phenylenediamine compound, and a thiocyanate compound. The compound preferably has an aromatic ring from the viewpoint of the compatibility of a film interface, and Spiro-OMeTAD, PTAA, or a phthalocyanine compound is particularly preferred.

### [Electron-transporting Layer]

In the photoelectric conversion element of the present invention, the electron-transporting layer 4 may be arranged between the second electrode 3 and the photoelectric conversion layer 5 as illustrated in Fig. 1.

A material for the electron-transporting layer 4 is not particularly limited, and examples thereof include an N-type conductive polymer, an N-type low-molecular-weight organic semiconductor, an N-type metal oxide, an N-type metal sulfide, a halogenated alkali metal, an alkali metal, and a surfactant. Specific examples thereof include a cyano group-containing polyphenylene vinylene, a boron-containing polymer, bathocuproine, bathophenanthroline, hydroxyquinolinatoaluminum, an oxadiazole compound, a benzimidazole compound, a naphthalenetetracarboxylic acid compound, a perylene derivative, a phosphine oxide compound, a phosphine sulfide compound, a fluoro group-containing phthalocyanine, titanium oxide, zinc oxide, indium oxide, tin oxide, gallium oxide, tin sulfide, indium sulfide, and zinc sulfide. In particular, tin oxide may be obtained through the reaction of tin(II) chloride, tin(IV) chloride, tin(II) chloride dihydrate, or tin(IV) chloride pentahydrate.

A preferred lower limit of the thickness of the electron-transporting layer 4 is 1 nm, and a preferred upper limit thereof is 2,000 nm. When such thickness is 1 nm or more, a hole can be sufficiently blocked, and when the thickness is 2,000 nm or less, the electron-transporting layer 4 is less liable to serve as a resistance at the time of the electron transportation, and hence the photoelectric conversion efficiency is improved. A more preferred lower limit of the thickness is 3 nm, a more preferred upper limit thereof is 1,000 nm, a still more preferred lower limit thereof is 5 nm, and a still more preferred upper limit thereof is 500 nm.

### <Application Examples>

Application examples of the present invention are directed to a photoelectric conversion apparatus, a moving body, and a building material.

### [Photoelectric Conversion Apparatus]

A photoelectric conversion apparatus of the present invention includes the above-mentioned photoelectric conversion element. The photoelectric conversion apparatus may be formed by using the plurality of photoelectric conversion elements of the present invention. When the plurality of photoelectric conversion elements are connected, such photoelectric conversion apparatus may also be referred to as "photoelectric conversion cell" or "photoelectric conversion module." Photoelectric conversion elements having different absorption wavelengths may be laminated as the photoelectric conversion elements to increase an output voltage. In addition, the photoelectric conversion apparatus includes the photoelectric conversion element of the present invention and an inverter. The inverter may be a converter for converting a DC voltage to an AC voltage. The photoelectric conversion apparatus may include an electricity storage unit connected to the photoelectric conversion element. The electricity storage unit is not limited as long as the electricity storage unit can store electricity. Examples thereof include a secondary battery using lithium ions, an all-solid-state battery, and an electric double layer capacitor.

### [Moving Body]

A moving body of the present invention includes the above-mentioned photoelectric conversion element. Fig. 2 is a perspective view for schematically illustrating a moving body including the photoelectric conversion element according to one embodiment of the present invention. A moving body 30 includes a photoelectric conversion element 31 of the present invention and a body 32 including the photoelectric conversion element 31. The photoelectric conversion element 31 is arranged on the position of the body 32 at which ambient light can be received. When the moving body 30 is an automobile, the photoelectric conversion element 31 may be arranged on a roof. Electric energy obtained by the photoelectric conversion element 31 may serve as the power of the moving body 30 or the power of any other electric equipment. Electric energy generated from the power of the moving body 30 may be used for the power of the photoelectric conversion element 31. When the moving body 30 is an automobile, friction energy generated with a brake may be converted into electric energy to be used for the control of the photoelectric conversion element 31.

The moving body 30 may be, for example, an automobile, a ship, an airplane, or a drone. The configuration of the body 32 of the moving body 30 is not particularly limited, but is preferably formed of a material having high strength.

### [Building Material]

A building material of the present invention includes the above-mentioned photoelectric conversion element. Fig. 3 is a perspective view for schematically illustrating a building material including the photoelectric conversion element according to one embodiment of the present invention. A building material 40 may be a roof of a building. The building material 40 of this embodiment includes a photoelectric conversion element 41 of the present invention, a protective member 42 for protecting the photoelectric conversion element 41, a heat dissipation member 43, and exteriors 44a and 44b.

The building material 40 of the present invention may include the heat dissipation member 43 having a thermal conductivity higher than that of the photoelectric conversion element 41. When the building material 40 is used for a roof or the like, the temperature of the photoelectric conversion element 41 may be increased by sunlight, and hence the photoelectric conversion efficiency may be reduced. The reduction of the photoelectric conversion efficiency can be suppressed by using the heat dissipation member 43. Examples of the heat dissipation member 43 include a metal, an alloy, a liquid metal, and a liquid resin.

In addition, the building material 40 of the present invention may include the exteriors 44a and 44b. The exterior 44a and the exterior 44b may show different colors, or may show the same color. The exterior 44a and the exterior 44b may be formed of the same member, or may be formed of different members. A paint or a transparent substrate may be used as each of the exteriors. An exterior having small light absorption and a high heat-shielding property is preferred.

### [Examples]

The present invention is described in more detail below by way of Examples and Comparative Examples. The present invention is by no means limited to the following Examples without departing from the gist thereof. In the description of the following Examples, the term "part(s)" is by mass unless otherwise specified.

### <Production of Particle 1>

### Step (1)

Under a nitrogen flow atmosphere, 5.46 parts of orthophthalonitrile and 45 parts of α-chloronaphthalene were loaded into a reaction kettle. After that, the mixture was heated so that its temperature was increased to 30°C, followed by the maintenance of the temperature. Next, 3.75 parts of gallium trichloride was loaded into the mixture at the temperature (30°C). The moisture concentration of the mixed liquid at the time of the loading was 150 ppm. After that, the temperature of the mixed liquid was increased to 200°C. Next, under a nitrogen flow atmosphere, the mixed liquid was subjected to a reaction at a temperature of 200°C for 4.5 hours, and was then cooled. The product was filtered when its temperature reached 150°C. The resultant filter residue was subjected to dispersion washing with N,N-dimethylformamide at a temperature of 140°C for 2 hours, and was then filtered. The resultant filter residue was washed with methanol, and was then dried to provide a chlorogallium phthalocyanine particle in a yield of 71%.

### Step (2)

4.65 Parts of the chlorogallium phthalocyanine particle was dissolved in 139.5 parts of concentrated sulfuric acid at a temperature of 10°C, and the solution was dropped into 620 parts of ice water under stirring so that the particle was reprecipitated, followed by filtration with a filter press under reduced pressure. At this time, No. 5C (manufactured by Advantec Toyo Kaisha, Ltd.) was used as a filter. The resultant wet cake (filter residue) was subjected to dispersion washing with 2% ammonia water for 30 minutes, and was then filtered with the filter press. Next, the resultant wet cake (filter residue) was subjected to dispersion washing with ion-exchanged water, and then its filtration with the filter press was repeated three times. Finally, the filter residue was freeze-dried to provide a hydroxygallium phthalocyanine particle (hydrous hydroxygallium phthalocyanine particle) having a solid content of 23 mass% in a yield of 71%. The hydroxygallium phthalocyanine particle was dried with a hyper-dry dryer (product name: HD-06R, frequency (oscillatory frequency): 2,455 MHz±15 MHz, manufactured by Biocon (Japan) Ltd.). Thus, a hydroxygallium phthalocyanine (OHGaPc) particle (crystal) having a water content of 1.0 mass% or less was obtained.

### Step (3)

5 Parts of the hydroxygallium phthalocyanine particle was mixed with 5 parts of an N-methylformamide solvent, and the mixture was subjected to dispersion treatment for 6 hours with a sand mill (TSG-1/4G-4U, manufactured by Igarashi Machine Production Co., Ltd. (currently AIMEX Co., Ltd.), disc diameter: 70 mm, number of discs: 5) containing 5 parts of glass beads, followed by filtration and drying to provide a particle 1 (specific gravity: 1.6).

### <Production of Resin Solution 1>

1.0 Gram of a polyvinyl acetal resin (product name: BM-2, manufactured by Sekisui Chemical Co., Ltd., glass transition temperature: 71°C) was dissolved in 19 g of 2-propanol under stirring for 24 hours to provide a resin solution 1.

### <Production of Resin Solution 2>

1.0 Gram of a polyvinyl acetal resin (product name: BX-1, manufactured by Sekisui Chemical Co., Ltd., glass transition temperature: 95°C) was dissolved in 19 g of 2-propanol under stirring for 24 hours to provide a resin solution 2.

### <Production of Resin Solution 3>

1.0 Gram of poly(methyl methacrylate) (PMMA, manufactured by Sigma-Aldrich LLC, glass transition temperature: 100°C) was dissolved in 19 g of chlorobenzene under stirring for 24 hours to provide a resin solution 3.

### <Production of Resin Solution 4>

1.0 Gram of poly(4-vinylpyridine) (glass transition temperature: 137°C) was dissolved in 19 g of 2-propanol under stirring for 24 hours to provide a resin solution 4.

### <Production of Resin Solution 5>

1.0 Gram of poly(3-hexylthiophene-2,5-diyl) (P3HT, glass transition temperature: 150°C<) was dissolved in 19 g of chlorobenzene under stirring for 24 hours to provide a resin solution 5.

### <Production of Resin Solution 6>

1.0 Gram of poly[9,9-bis-(2-ethylhexyl)-9H-fluorene-2,7-diyl] (glass transition temperature: 45°C) was dissolved in 19 g of chlorobenzene under stirring for 24 hours to provide a resin solution 6.

### (Example 1)

### [Formation of Electron-transporting Layer]

A glass substrate with ITO was washed, and a tin(II) oxide colloidal solution (15% water dispersion, manufactured by Alfa Aesar) diluted fivefold was applied thereonto by spin coating, followed by heating at 150°C for 30 minutes to form an electron-transporting layer as a thin film having a thickness of 16 nm.

### [Formation of Photoelectric Conversion Layer]

1.1 Grams of lead iodide and 0.39 g of methylammonium iodide were dissolved in 0.36 g of N,N-dimethylformamide and 1.45 g of dimethyl sulfoxide to prepare a photoelectric conversion layer coating liquid. The coating liquid was applied onto the electron-transporting layer by spin coating to form a photoelectric conversion layer formed of MAPbI₃ and having a thickness of 500 nm.

### [Preparation of Composition and Formation of Charge-transporting Layer]

0.1 Gram of the particle 1 and 0.01 g of a calixarene compound (mixed product of the calixarene compounds represented by the formulae [C-1] to [C-4]) were mixed with 10.6 g of 2-propanol, 11 g of beads (zirconia beads, TORAYCERAM (trademark) zirconia beads, 0.3 mm) were loaded into the mixture, and dispersion was performed with a paint shaker (manufactured by Toyo Seiki Seisaku-sho, Ltd.) for 3 hours to provide a dispersion liquid. After that, 0.2 g of the resin solution 1 was added to the dispersion liquid (post-addition), and dispersion was performed with the paint shaker again for 4 hours to prepare a charge-transporting layer coating liquid. The charge-transporting layer coating liquid was applied onto the photoelectric conversion layer by spin coating to form a charge-transporting layer having a thickness of 180 nm.

### [Introduction of Second Charge-transporting Layer]

0.15 Gram of Spiro-OMeTAD serving as a material for a second charge-transporting layer was dissolved in 2.2 g of chlorobenzene. 36 Microliters of an acetonitrile solution obtained by dissolving 0.2 g of lithium bis(trifluoromethanesulfonyl)imide in 0.3 g of acetonitrile and 60 µL of 4-tert-butylpyridine (TBP) were added to the chlorobenzene solution, and the contents were mixed. Further, 58 µL of an acetonitrile solution obtained by dissolving 0.11 g of [tris(2-(1H-pyrazol-1-yl)-4-tert-butylpyridine)cobalt(III) tris(bis(trifluoromethylsulfonyl)imide)] in 0.3 g of acetonitrile was mixed thereinto to prepare a material solution for a second charge-transporting layer. The material solution was applied onto the above-mentioned charge-transporting layer by a spin coating method to form a second charge-transporting layer having a thickness of 150 nm. It was recognized that the film had a ratio of the intensity of a peak in the range of a Bragg angle 2θ of 7.6 to 8.6° with respect to the total peak intensity of 0.0 to 30.0° of 0.023 in an X-ray diffraction spectrum using CuKα rays.

### [Formation of First Electrode]

A gold electrode having a thickness of 80 nm and an area of 0.09 cm² was formed on the second charge-transporting layer by a vacuum vapor deposition method. Thus, a photoelectric conversion element was obtained.

### [Analysis of Particle Diameter of Composition]

The particle diameter of the pigment particle of the composition serving as the charge-transporting layer coating liquid was determined by measuring a Z-average particle diameter with the above-mentioned Zetasizer Nano ZS (manufactured by Malvern Panalytical Ltd.). In an initial stage, the composition was subjected to the measurement within 3 days after its preparation. With regard to the measurement after 3 months, the composition, which had been stored stationarily in a sealed container after its preparation, was subjected to the measurement after the container had been shaken well to be brought into a state in which no precipitate was observed at its bottom.

### [Photoelectric Conversion Efficiency Evaluation]

A power supply (236 model, manufactured by Keithley Instruments) was connected between the electrodes of the photoelectric conversion element produced in Example 1, and its photoelectric conversion efficiency was measured by: irradiating the element with constant light through use of a solar simulator (manufactured by Yamashita Denso Corporation) having an intensity of 100 mW/cm²; and measuring the generated current and voltage. The results are shown in Table 2.

### (Comparative Example 1)

A composition and a photoelectric conversion element were obtained in the same manner as in Example 1 except for a method of preparing the composition serving as the charge-transporting layer coating liquid. The method of preparing the composition is as described below. 0.1 Gram of the particle 1, 0.00468 g (0.15 times with respect to a resin) of a calixarene compound (mixed product of the compounds represented by the formulae [C-1] to [C-4]), 2.2 g of cyclohexanone, and 0.03 g of a BX-1 resin were mixed. Beads (glass beads, 1 mm) were loaded into the mixture, and dispersion was performed with a paint shaker (manufactured by Toyo Seiki Seisaku-sho, Ltd.) for 6 hours to provide a dispersion liquid. After that, 2.2 g of ethyl acetate was added to the dispersion liquid to dilute the liquid. It was recognized that the film had a ratio of the intensity of a peak in the range of a Bragg angle 2θ of 7.6 to 8.6° with respect to the total peak intensity of 0.0 to 30.0° of 0.015 in an X-ray diffraction spectrum using CuKα rays.

### (Examples 2 to 29 and Comparative Examples 2 to 6)

Compositions and photoelectric conversion elements are each produced and evaluated in the same manner as in Example 1 except that, in the preparation of the composition serving as the charge-transporting layer coating liquid, the kind of the calixarene compound, the ratio of the calixarene compound with respect to the resin, the kind of the cyclic conjugated compound, the kind of the resin, the kind of the solvent, the timing of the addition of the resin, the ratio of the pigment with respect to the resin, and the presence or absence of the formation of the second charge-transporting layer in the formation of the element are changed. The results are shown in Table 2.

The kinds of calixarene compounds (the kinds of dispersants) [C-5] to [C-9] are shown below.

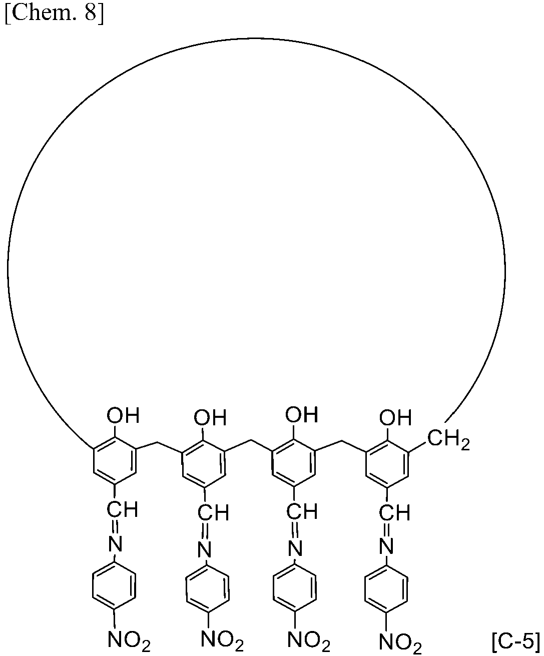

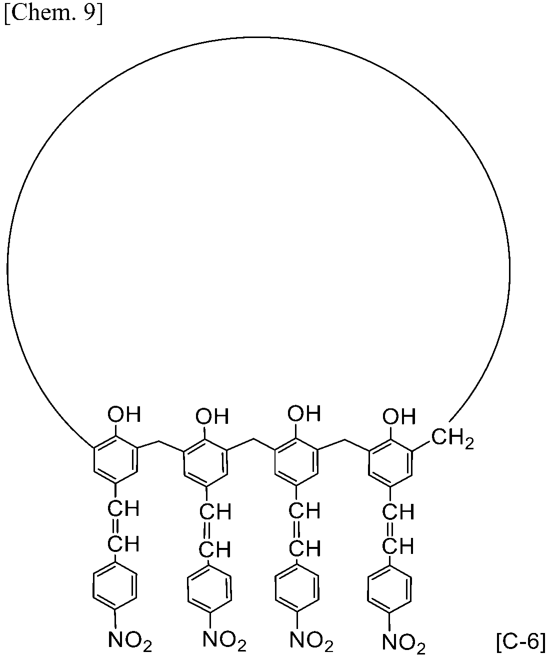

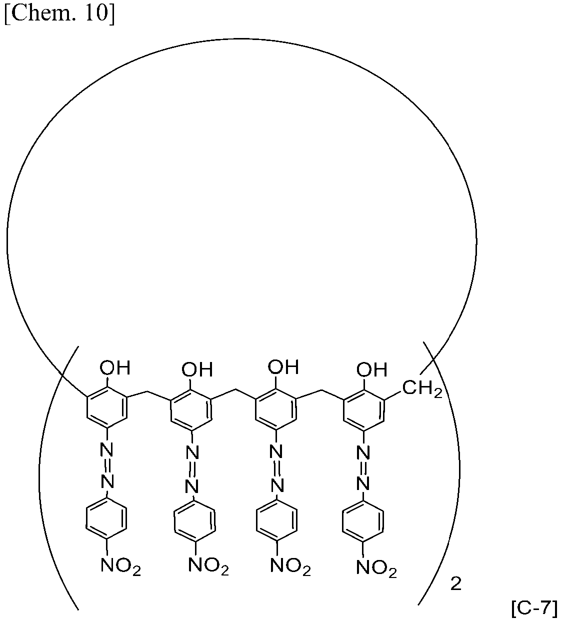

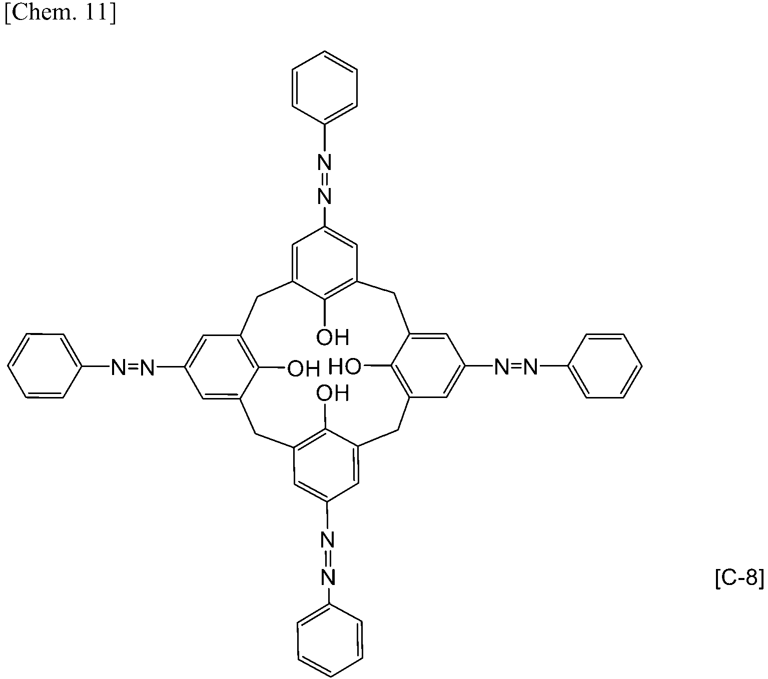

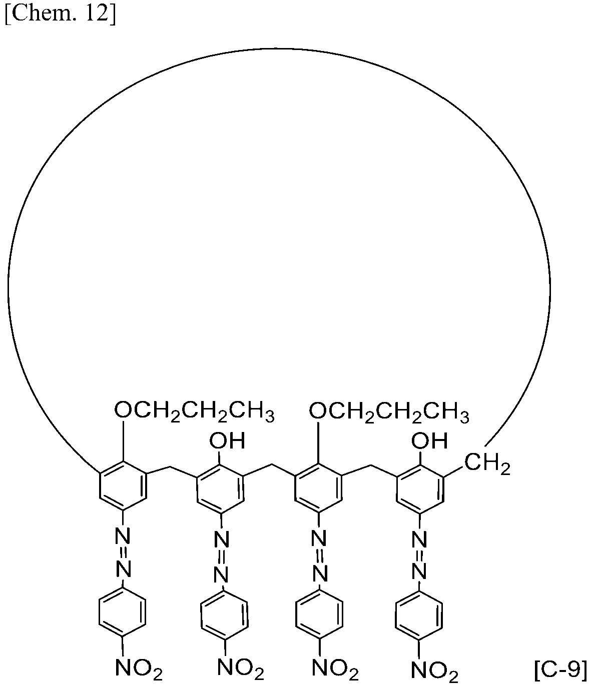

The term "pre-addition" in the section "Resin addition timing" in Table 2 in Example 25 and Comparative Example 2 means that, in the preparation of the composition, the resin solution 1 is added from the start together with the particle 1, the calixarene compound, and the solvent, and dispersion is performed with a paint shaker for 7 hours to provide a charge-transporting layer coating liquid.

**[Table 2]**

| Example | Composition configuration | | | |
|---|---|---|---|---|
| | Kind of pigment (cyclic conjugated compound) | Kind of dispersant | Dispersant/ pigment weight ratio | Kind of resin |
| 1 | Hydroxygallium phthalocyanine | Mixed product of [C-1] to [C-4] | 0.10 times | BM2 |
| 2 | Hydroxygallium phthalocyanine | Mixed product of [C-1] to [C-4] | 0.20 times | BM2 |
| 3 | Hydroxygallium phthalocyanine | Mixed product of [C-1] to [C-4] | 0.02 times | BM2 |
| 4 | Hydroxygallium phthalocyanine | [C-5] | 0.10 times | BM2 |
| 5 | Hydroxygallium phthalocyanine | [C-6] | 0.10 times | BM2 |
| 6 | Hydroxygallium phthalocyanine | [C-7] | 0.10 times | BM2 |
| 7 | Hydroxygallium phthalocyanine | [C-8] | 0.10 times | BM2 |
| 8 | Hydroxygallium phthalocyanine | [C-9] | 0.10 times | BM2 |
| 9 | 2,3,7,8,12,13,17,18-Octaphenyl-5,10,15,20-tetraazaporphyrin | Mixed product of [C-1] to [C-4] | 0.10 times | BM2 |
| 10 | Titanyl phthalocyanine | Mixed product of [C-1] to [C-4] | 0.10 times | BM2 |
| 11 | Phthalocyanine | Mixed product of [C-1] to [C-4] | 0.10 times | BM2 |
| 12 | Aluminum phthalocyanine | Mixed product of [C-1] to [C-4] | 0.10 times | BM2 |
| 13 | Iron phthalocyanine | Mixed product of [C-1] to [C-4] | 0.10 times | BM2 |
| 14 | Dichlorosilicon phthalocyanine | Mixed product of [C-1] to [C-4] | 0.10 times | BM2 |
| 15 | Copper phthalocyanine | Mixed product of [C-1] to [C-4] | 0.10 times | BM2 |
| 16 | Chlorogallium phthalocyanine | Mixed product of [C-1] to [C-4] | 0.10 times | BM2 |
| 17 | Hydroxygallium phthalocyanine | Mixed product of [C-1] to [C-4] | 0.10 times | BX1 |
| 18 | Hydroxygallium phthalocyanine | [C-9] | 0.10 times | BX1 |
| 19 | Hydroxygallium phthalocyanine | Mixed product of [C-1] to [C-4] | 0.10 times | PMMA |
| 20 | Hydroxygallium phthalocyanine | Mixed product of [C-1] to [C-4] | 0.10 times | Poly(4-vinylpyridine) |
| 21 | Hydroxygallium phthalocyanine | Mixed product of [C-1] to [C-4] | 0.10 times | P3HT |
| 22 | Hydroxygallium phthalocyanine | Mixed product of [C-1] to [C-4] | 0.10 times | Poly[9,9-bis-(2-ethylhexyl)-9H-fluorene-2,7-diyl] |
| 23 | Hydroxygallium phthalocyanine | Mixed product of [C-1] to [C-4] | 0.10 times | BM2 |
| 24 | Hydroxygallium phthalocyanine | Mixed product of [C-1] to [C-4] | 0.10 times | BM2 |
| 25 | Hydroxygallium phthalocyanine | Mixed product of [C-1] to [C-4] | 0.10 times | BM2 |
| 26 | Hydroxygallium phthalocyanine | Mixed product of [C-1] to [C-4] | 0.10 times | BM2 |
| 27 | Hydroxygallium phthalocyanine | Mixed product of [C-1] to [C-4] | 0.10 times | BM2 |
| 28 | Hydroxygallium phthalocyanine | Mixed product of [C-1] to [C-4] | 0.20 times | BM2 |
| 29 | Hydroxygallium phthalocyanine | Mixed product of [C-1] to [C-4] | 0.05 times | BM2 |
| Comparative Example 1 | Hydroxygallium phthalocyanine | Mixed product of [C-1] to [C-4] | 0.047 times | BX1 |
| Comparative Example 2 | Hydroxygallium phthalocyanine | Mixed product of [C-1] to [C-4] | 0.015 times | BM2 |
| Comparative Example 3 | Hydroxygallium phthalocyanine | Mixed product of [C-1] to [C-4] | 0.015 times | BM2 |
| Comparative Example 4 | Hydroxygallium phthalocyanine | Mixed product of [C-1] to [C-4] | 0.015 times | - |
| Comparative Example 5 | 2,3,7,8,12,13,17,18-Octaphenyl-5,10,15,20-tetraazaporphyrin | Mixed product of [C-1] to [C-4] | 0.015 times | BM2 |
| Comparative Example 6 | Titanyl phthalocyanine | Mixed product of [C-1] to [C-4] | 0.015 times | BM2 |

**[Table 2] continued**

| Example | Composition configuration | | | | |
|---|---|---|---|---|---|
| | Resin Tg | Dispersant/res in weight ratio | Pigment/resin weight ratio | Solvent | Solvent vapor pressure (20°C) kPa |
| 1 | 71°C | 1.00 times | 10 times | 2-Propanol | 4.3 |
| 2 | 71°C | 2.00 times | 10 times | 2-Propanol | 4.3 |
| 3 | 71°C | 0.20 times | 10 times | 2-Propanol | 4.3 |
| 4 | 71°C | 1.00 times | 10 times | 2-Propanol | 4.3 |
| 5 | 71°C | 1.00 times | 10 times | 2-Propanol | 4.3 |
| 6 | 71°C | 1.00 times | 10 times | 2-Propanol | 4.3 |
| 7 | 71°C | 1.00 times | 10 times | 2-Propanol | 4.3 |
| 8 | 71°C | 1.00 times | 10 times | 2-Propanol | 4.3 |
| 9 | 71°C | 1.00 times | 10 times | 2-Propanol | 4.3 |
| 10 | 71°C | 1.00 times | 10 times | 2-Propanol | 4.3 |
| 11 | 71°C | 1.00 times | 10 times | 2-Propanol | 4.3 |
| 12 | 71°C | 1.00 times | 10 times | 2-Propanol | 4.3 |
| 13 | 71°C | 1.00 times | 10 times | 2-Propanol | 4.3 |
| 14 | 71°C | 1.00 times | 10 times | 2-Propanol | 4.3 |
| 15 | 71°C | 1.00 times | 10 times | 2-Propanol | 4.3 |
| 16 | 71°C | 1.00 times | 10 times | 2-Propanol | 4.3 |
| 17 | 95°C | 1.00 times | 10 times | 2-Propanol | 4.3 |
| 18 | 95°C | 1.00 times | 10 times | 2-Propanol | 4.3 |
| 19 | 100° C | 1.00 times | 10 times | 2-Propanol | 4.3 |
| 20 | 137° C | 1.00 times | 10 times | 2-Propanol | 4.3 |
| 21 | 150° C< | 1.00 times | 10 times | 2-Propanol | 4.3 |
| 22 | 45°C | 1.00 times | 10 times | 2-Propanol | 4.3 |
| 23 | 71°C | 1.00 times | 10 times | 1-Butanol | 0.58 |
| 24 | 71°C | 1.00 times | 10 times | Cyclohexanol:benzyl alcohol=1:1 | 0.06 |
| 25 | 71°C | 1.00 times | 10 times | Dipropylene glycol | 0.05 |
| 26 | 71°C | 1.00 times | 10 times | Methyl benzoate | 0.05 |
| 27 | 71°C | 1.00 times | 10 times | 2-Propanol | 4.3 |
| 28 | 71°C | 1.00 times | 5 times | 2-Propanol | 4.3 |
| 29 | 71°C | 1.00 times | 20 times | 2-Propanol | 4.3 |
| Comparative Example 1 | 95°C | 0.15 times | 21 times | Cyclohexanone:ethyl acetate=1:1 | 5.3 |
| Comparative Example 2 | 71°C | 0.15 times | 10 times | 2-Propanol | 4.3 |
| Comparative Example 3 | 71°C | 0.15 times | 10 times | 2-Propanol | 4.3 |
| Comparative Example 4 | - | - | - | 2-Propanol | 4.3 |
| Comparative Example 5 | 71°C | 0.15 times | 10 times | 2-Propanol | 4.3 |
| Comparative Example 6 | 71°C | 0.15 times | 10 times | 2-Propanol | 4.3 |

**[Table 2] continued**

| Example | Composition production method | Composition physical properties | | Element characteristic |
|---|---|---|---|---|
| | Resin addition timing | Initial particle diameter [nm] | Particle diameter after 3 months [nm] | Photoelectric conversion efficiency [%] |
| 1 | Post-addition | 1.8×10² | 1.9×10² | 18.0 |
| 2 | Post-addition | 1.5×10² | 1.5×10² | 18.5 |
| 3 | Post-addition | 3.0×10² | 4.1×10² | 17.5 |
| 4 | Post-addition | 2.0×10² | 2.0×10² | 17.8 |
| 5 | Post-addition | 2.1×10² | 2.1×10² | 17.8 |
| 6 | Post-addition | 2.5×10² | 2.8×10² | 17.5 |
| 7 | Post-addition | 2.9×10² | 3.3×10² | 17.1 |
| 8 | Post-addition | 2.3×10² | 2.3×10² | 17.8 |
| 9 | Post-addition | 2.8×10² | 3.0×10² | 16.5 |
| 10 | Post-addition | 2.3×10² | 2.4×10² | 17.0 |
| 11 | Post-addition | 2.4×10² | 2.5×10² | 16.8 |
| 12 | Post-addition | 2.3×10² | 2.4×10² | 16.9 |
| 13 | Post-addition | 2.2×10² | 2.3×10² | 17.0 |
| 14 | Post-addition | 2.4×10² | 2.5×10² | 16.8 |
| 15 | Post-addition | 2.3×10² | 2.4×10² | 16.3 |
| 16 | Post-addition | 2.0×10² | 2.0×10² | 17.5 |
| 17 | Post-addition | 2.2×10² | 2.3×10² | 17.9 |
| 18 | Post-addition | 2.4×10² | 2.5×10² | 17.8 |
| 19 | Post-addition | 2.8×10² | 3.0×10² | 17.6 |
| 20 | Post-addition | 2.9×10² | 3.0×10² | 17.5 |
| 21 | Post-addition | 2.9×10² | 3.1×10² | 17.4 |
| 22 | Post-addition | 3.3×10² | 3.5×10² | 16.8 |
| 23 | Post-addition | 2.0×10² | 2.2×10² | 17.9 |
| 24 | Post-addition | 2.5×10² | 2.8×10² | 17.8 |
| 25 | Post-addition | 2.9×10² | 3.3×10² | 17.3 |
| 26 | Post-addition | 3.5×10² | 4.1×10² | 16.8 |
| 27 | Pre-addition | 3.5×10² | 4.2×10² | 17 |
| 28 | Post-addition | 2.2×10² | 2.4×10² | 17.5 |
| 29 | Post-addition | 2.7×10² | 3.0×10² | 17 |
| Comparative Example 1 | Pre-addition | 2.4×10² | 2.5×10² | 13.5 |
| Comparative Example 2 | Post-addition | 4.0×10² | 5.1×10² | 15.8 |
| Comparative Example 3 | Pre-addition | 4.9×10² | 6.0×10² | 15.3 |
| Comparative Example 4 | Post-addition | 1.7×10² | 5.5×10² | 14.3 |
| Comparative Example 5 | Post-addition | 4.7×10² | 5.5×10² | 14.9 |
| Comparative Example 6 | Post-addition | 4.4×10² | 5.6×10² | 15.1 |

The present invention is not limited to the embodiments described above, and various changes and modifications may be made without departing from the spirit and scope of the present invention. The following claims are appended hereto in order to make the scope of the present invention public.

The present application claims priority based on Japanese Patent Application No. 2023-184761 filed on October 27, 2023, Japanese Patent Application No. 2023-184756 filed on October 27, 2023, Japanese Patent Application No. 2023-184750 filed on October 27, 2023, Japanese Patent Application No. 2023-216294 filed on December 21, 2023, Japanese Patent Application No. 2023-216296 filed on December 21, 2023, Japanese Patent Application No. 2023-216299 filed on December 21, 2023, Japanese Patent Application No. 2024-022244 filed on February 16, 2024, Japanese Patent Application No. 2024-022251 filed on February 16, 2024, Japanese Patent Application No. 2024-022246 filed on February 16, 2024, Japanese Patent Application No. 2024-177315 filed on October 9, 2024, and Japanese Patent Application No. 2024-186707 filed on October 23, 2024, and the entire contents thereof are incorporated herein by reference.

### [Reference Signs List]

1 photoelectric conversion element
2 substrate
3 second electrode
4 electron-transporting layer
5 photoelectric conversion layer
6 charge-transporting layer
7 first electrode
30 moving body
31, 41 photoelectric conversion element
32 body
40 building material
42 protective member
43 heat dissipation member
44a, 44b exterior

## Claims

1. A composition comprising:
a pigment that is a cyclic conjugated compound in which a plurality of pyrrole rings are bonded by conjugated bonds;
a dispersant;
a resin; and
a solvent,
wherein the dispersant is a calixarene compound represented by the following formula [A], and
wherein a mass of the dispersant in the composition is 0.20 times or more with respect to a mass of the resin in the composition: 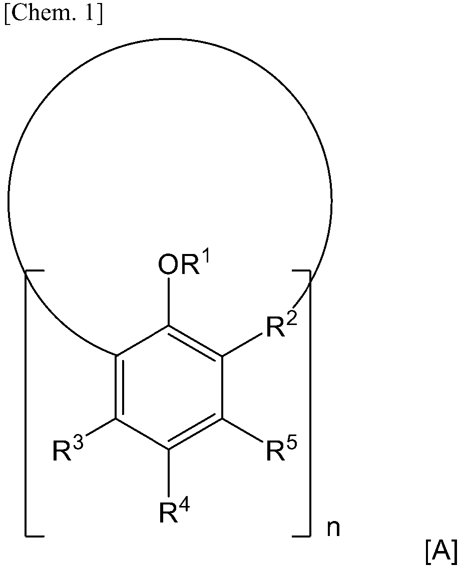 in the formula [A], R¹ to R⁵ are as follows each independently in each repeating unit and each independently for "n" repeating units: R¹ represents a hydrogen atom or an alkyl group; R² represents a substituted or unsubstituted alkylene group; and R³ to R⁵ each represent a hydrogen atom, a substituted or unsubstituted alkyl group, a substituted or unsubstituted -Y-Ar group, a substituted or unsubstituted aromatic hydrocarbon group, or a substituted or unsubstituted heterocyclic group, and at least one of R³ to R⁵ represents a substituted or unsubstituted -Y-Ar group, where -Y- of the -Y-Ar group represents -CH=N-, -CH=CH-, or -N=N-, and Ar represents a substituted or unsubstituted aromatic hydrocarbon group, or a substituted or unsubstituted heterocyclic group, and "n" represents an integer of 3 to 20.

2. The composition according to claim 1, wherein in the formula [A], "n" represents 4 to 8.

3. The composition according to claim 1 or 2, wherein in the formula [A], R⁴ represents a nitrophenylazo group or a dinitrophenylazo group each independently for "n" repeating units.

4. The composition according to any one of claims 1 to 3, wherein the calixarene compound represented by the formula [A] has a molecular weight of 10,000 or less.

5. The composition according to any one of claims 1 to 4, wherein the calixarene compound represented by the formula [A] includes at least one selected from the group consisting of compounds represented by the following formulae [C-1], [C-2], [C-3], and [C-4].

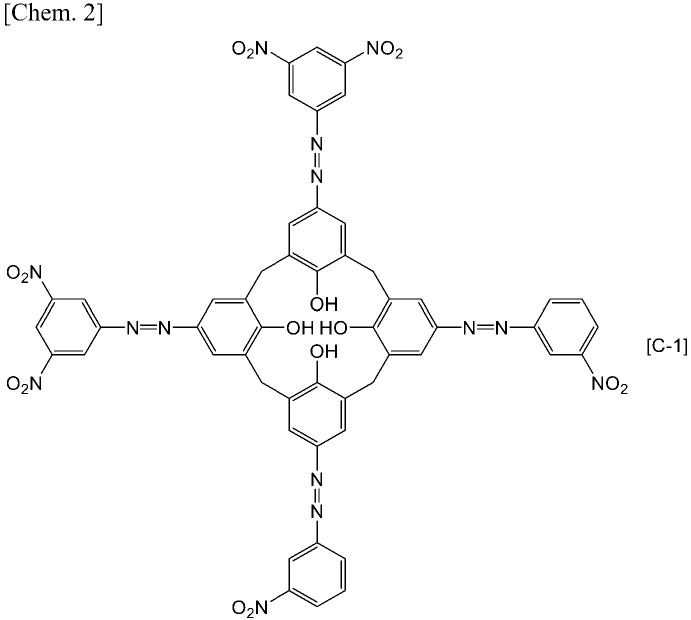

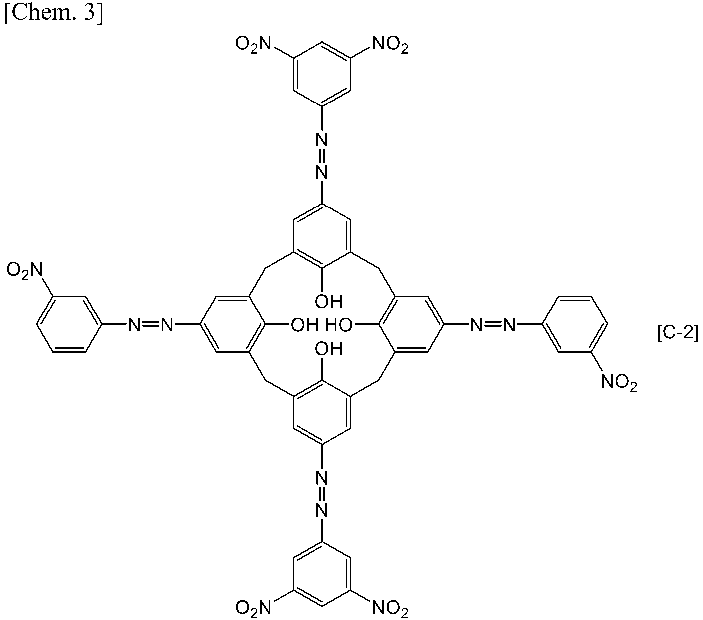

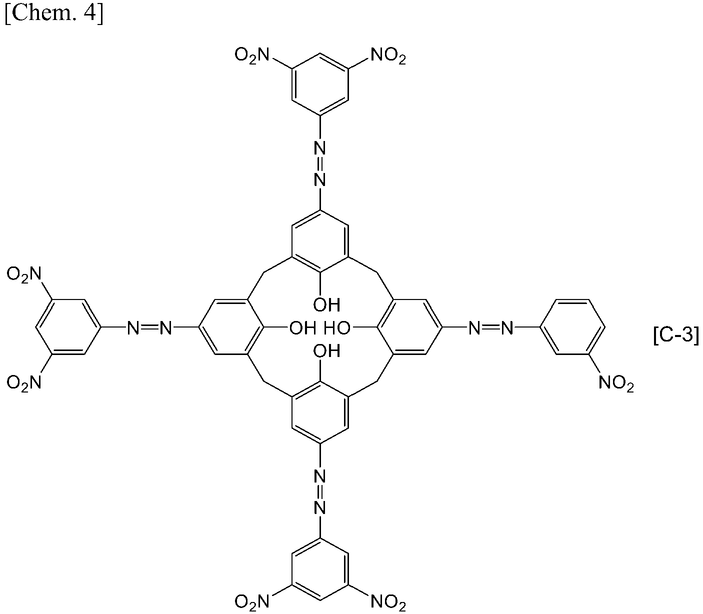

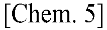

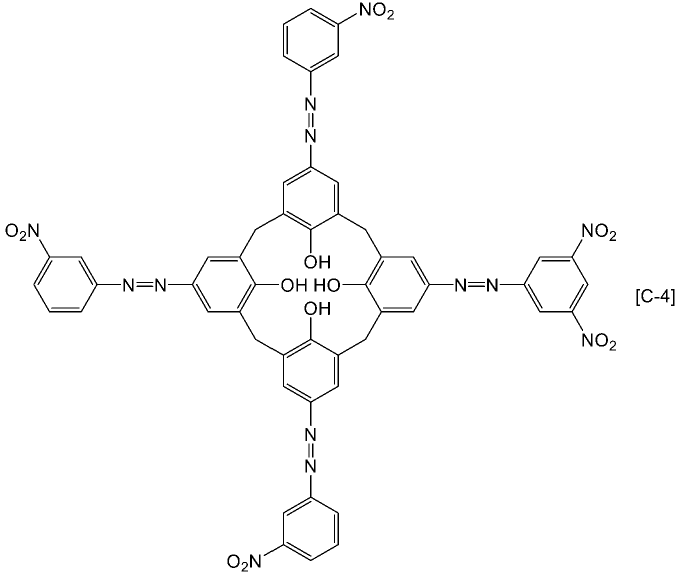

6. The composition according to any one of claims 1 to 5, wherein the pigment that is a cyclic conjugated compound in which a plurality of pyrrole rings are bonded by conjugated bonds is a phthalocyanine compound.

7. The composition according to claim 6, wherein a central element of the phthalocyanine compound is metal-free, or at least one of gallium, aluminum, titanium, iron, or silicon.

8. The composition according to claim 6, wherein the phthalocyanine compound is a gallium phthalocyanine compound.

9. The composition according to claim 8, wherein the gallium phthalocyanine compound is hydroxygallium phthalocyanine.

10. The composition according to any one of claims 1 to 9, wherein the resin is a resin having a Lewis basic functional group.

11. The composition according to any one of claims 1 to 10, wherein the resin has at least one functional group selected from the group consisting of: a hydroxy group; a carbonyl group; an ether bond; an ester bond; a pyridyl group; and a thienyl group.

12. The composition according to any one of claims 1 to 11, wherein the resin has a glass transition temperature (Tg) of 95°C or less.

13. The composition according to claim 11 or 12, wherein the resin is a polyvinyl acetal resin or a polyvinyl butyral resin.

14. The composition according to any one of claims 1 to 13, wherein the solvent has a vapor pressure (20°C) of 0.06 kPa or more.

15. The composition according to any one of claims 1 to 14, wherein the mass of the dispersant in the composition is 0.01 to 0.50 times with respect to a mass of the pigment in the composition.

16. The composition according to any one of claims 1 to 15, wherein a mass of the pigment in the composition is 5 to 20 times with respect to the mass of the resin in the composition.

17. A method of preparing a composition including a pigment that is a cyclic conjugated compound in which a plurality of pyrrole rings are bonded by conjugated bonds, a dispersant, a resin, and a solvent,
wherein the dispersant is a calixarene compound represented by the following formula [A],
wherein a mass of the dispersant in the composition is 0.20 times or more with respect to a mass of the resin in the composition, and
wherein the method comprises dispersing the pigment and the dispersant with the solvent to provide a dispersion liquid, and then adding the resin to the dispersion liquid to disperse the pigment, the dispersant, and the resin again: 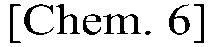 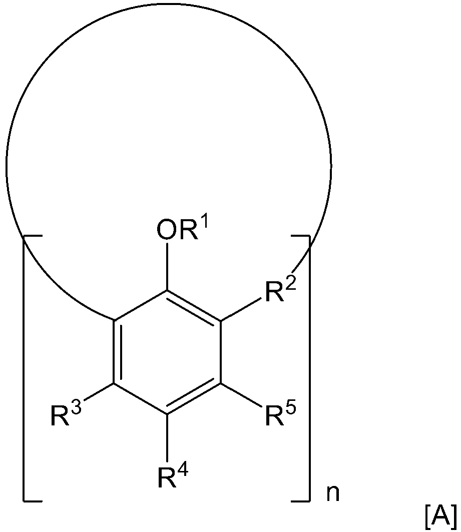 in the formula [A], R¹ to R⁵ are as follows each independently in each repeating unit and each independently for "n" repeating units: R¹ represents a hydrogen atom or an alkyl group; R² represents a substituted or unsubstituted alkylene group; and R³ to R⁵ each represent a hydrogen atom, a substituted or unsubstituted alkyl group, a substituted or unsubstituted -Y-Ar group, a substituted or unsubstituted aromatic hydrocarbon group, or a substituted or unsubstituted heterocyclic group, and at least one of R³ to R⁵ represents a substituted or unsubstituted -Y-Ar group, where -Y- of the -Y-Ar group represents -CH=N-, -CH=CH-, or -N=N-, and Ar represents a substituted or unsubstituted aromatic hydrocarbon group, or a substituted or unsubstituted heterocyclic group, and "n" represents an integer of 3 to 20.

18. The composition according to any one of claims 1 to 16, wherein the pigment has a particle diameter of 1.0×10¹ to 5.0×10² nm.

19. A photoelectric conversion element comprising:
a first electrode;
a photoelectric conversion layer containing a crystal having a perovskite structure;
a charge-transporting layer; and
a second electrode,
wherein the photoelectric conversion element comprises, between the photoelectric conversion layer and the first electrode, a charge-transporting layer formed by using the composition of any one of claims 1 to 16 or 18.

20. The photoelectric conversion element according to claim 19, wherein the photoelectric conversion element further comprises a second charge-transporting layer between the first electrode and the charge-transporting layer.
